# EUROPEAN PATENT SPECIFICATION

(11) **EP 3 150 710 B1**
(45) Date of publication and mention of the grant of the patent: **11.05.2022**
(21) Application number: 14886152.9
(22) Date of filing: 24.03.2014
(51) Int. Cl.: C12P 7/40, C12P 7/54, C12N 1/21, C07D 207/263, C07D 307/33, C12N 15/52, C12P 13/06

(54) **METHOD FOR PREPARING HOMOSERINE LACTONE AND ORGANIC ACID FROM MICROORGANISM-DERIVED O-ACYL HOMOSERINE**
VERFAHREN ZUR HERSTELLUNG VON HOMOSERINLACTON UND ORGANISCHER SÄURE AUS MIT MIKROORGANISMEN HERGESTELLTEM O-ACYL-HOMOSERIN
PROCÉDÉ DE PRÉPARATION DE L'HOMOSÉRINE LACTONE ET D'ACIDE ORGANIQUE À PARTIR D'O-ACYLHOMOSÉRINE ISSUE DE MICRO-ORGANISMES

(30) Priority: 20.03.2014 KR 20140032512
(43) Date of publication of application: 05.04.2017
(73) Proprietor: CJ Cheiljedang Corporation, Seoul 04560 (KR)
(72) Inventor: YANG, Young Lyeol, Seoul 06317 (KR); LEE, Han Won, Seoul 05048 (KR); GOH, Young Hyong, Tongyeong-si Gyeongsangnam-do 53049 (KR); KIM, So Young, Gwacheon-si Gyeonggi-do 13803 (KR); SHIN, Yong Uk, Gyeonggi-do 16898 (KR); UM, Hye Won, Suwon-si Gyeonggi-do 16507 (KR); CHANG, Jin Sook, Gyeonggi-do 16532 (KR); JHON, Sung Hoo, Seoul 07525 (KR)
(74) Representative: Dehns
(86) International application number: PCT/KR2014/002467
(87) International publication number: WO 2015/141886

(56) References cited:
- EP-A1- 2 290 051
- EP-A2- 2 292 783
- WO-A1-2008/013432
- JP-A- 2001 002 668
- JP-A- 2012 143 183
- JP-B2- 5 383 203
- KR-A- 20080 011 132
- KR-A- 20130 006 464
- US-A- 3 114 701
- US-A1- 2009 253 187
- US-A1- 2013 225 877
- US-A1- 2013 273 615
- SAMUEL NATELSON: "Specific assay for homoserine and its lactone in Pisum sativum. Preparation of homoserine hydroxamic acid", MICROCHEMICAL JOURNAL, vol. 27, no. 4, December 1982 (1982-12), pages 466-483, XP055404992, US ISSN: 0026-265X, DOI: 10.1016/0026-265X(82)90002-9
- AKIHIKO NAGAO ET AL: "Synthesis of O-Acyl-L-Homoserine by lipase", JOURNAL OF THE AMERICAN OIL CHEMISTS' SOCIETY (JA, SPRINGER, DE, vol. 66, no. 5, May 1989 (1989-05), pages 710-713, XP008178149, ISSN: 0003-021X, DOI: 10.1007/BF02669958
- KASE H ET AL: "PRODUCTION OF O-ACETYL-L-HOMOSERINE BY METHIONINE ANALOG-RESISTANT MUTANTS AND REGULATION OF HOMOSERINE-O-TRANSACETYLASE IN CORYNEBACTERIUM GLUTAMICUM", AGRICULTURAL AND BIOLOGICAL CHEMISTRY, AGRICULTURAL CHEMICAL SOCIETY OF JAPAN, JP, vol. 38, no. 10, October 1974 (1974-10), pages 2021-2030, XP001038200, ISSN: 0002-1369
- SADAMU NAGAI ET AL: "Acetylhomoserine an intermediate in the fungal biosynthesis of methionine", THE JOURNAL OF BIOLOGICAL CHEMISTRY, vol. 242, 10 September 1967 (1967-09-10), pages 3884-3895, XP055404988,
- UNG GI HONG ET AL: "Hydrogenation of succinic acid to -butyrolactone (GBL) over ruthenium catalyst supported on surfactant-templated mesoporous carbon", JOURNAL OF INDUSTRIAL AND ENGINEERING CHEMISTRY, THE KOREAN SOCIETY OF INDUSTRIAL AND ENGINEERING CHEMISTRY, KOREA, vol. 18, no. 1, 22 May 2011 (2011-05-22), pages 462-468, XP028434483, ISSN: 1226-086X, DOI: 10.1016/J.JIEC.2011.11.054 [retrieved on 2011-11-07]
- CLARA DELHOMME ET AL: "Succinic acid from renewable resources as a C4 building-block chemical-a review of the catalytic possibilities in aqueous media", GREEN CHEMISTRY, vol. 11, no. 1, 2009, page 13, XP055160805, ISSN: 1463-9262, DOI: 10.1039/B810684C
- None
- VOELKERT E ET AL: "Determination of homoserine as the lactone", ANALYTICAL BIOCHEMISTRY, ACADEMIC PRESS, AMSTERDAM, NL, vol. 34, no. 1, 1 March 1970 (1970-03-01), pages 131-137, XP024816357, ISSN: 0003-2697, DOI: 10.1016/0003-2697(70)90093-X [retrieved on 1970-03-01]

## Description

### BACKGROUND OF THE INVENTION

### Field of the Invention

The present invention relates to a method for producing bio-based homoserine lactone and bio-based organic acid and its derivatives.

### Description of the Prior Art

Raw material for producing industrially useful gamma-butyrolactone, 1,4-butanediol, tetrahydrofuran and the like, are mostly petrochemicals, for example, maleic anhydride, anhydrous succinic acid, acetylene, butadiene and the like.

Thus, there have recently been attempts to use environmental-friendly bio-based materials which can solve environmental concerns including the emission of pollutants and the exhaustion of natural resources and can be renewable, as substitutes for conventional petrochemicals, raw material for producing gamma-butyrolactone, 1,4-butanediol, tetrahydrofuran and the like.

For example, biodegradable polybutylene succinate can be produced by esterifying 1,4-butanediol with succinic acid and polycondensing the resulting oligomer by transesterification, and polybutylene terephthalate can be produced by esterification of 1,4-butanediol with terephthalic acid.

In recent years, succinic acid has been produced from biomass by direct microbial fermentation and has been commercially used for the production of tetrahydrofuran, 1,4-butanediol, gamma-butyrolactone and the like (Bio-Amber Inc.). US Patent Publication No. 2011/0159572 discloses a microbial organisms containing a 1,4-butanediol (BDO) pathway comprising at least one exogenous nucleic acid encoding a 1,4-butanediol (BDO) pathway enzyme expressed in a sufficient amount to produce 1,4-butanediol.

WO-A- 2008/013432, US-A1- 2013/273615, EP-A- 2 290 051 teach methods for producing O-acetyl homoserine by culturing microorganisms.

However, there were no reports yet of the use of bio-based homoserine lactone and bio-based organic acid, obtained by chemical conversion of O-acyl homoserine produced by a microorganism, as a starting material for the synthesis of industrially useful 1,4-butanediol, gamma-butyrolactone, tetrahydrofuran and the like.

### SUMMARY OF THE INVENTION

It is an object of the present invention to provide the novel use of bio-based O-acyl homoserine which can solve environmental concerns including the emission of pollutants and the exhaustion of natural resources and can be renewable, as substitutes for conventional petrochemicals, raw material for producing gamma-butyrolactone, 1,4-butanediol, tetrahydrofuran and the like.

More specifically, an object of the present invention is to provide a method of synthesizing industrially useful 1,4-butanediol, gamma-butyrolactone, tetrahydrofuran and the like, using bio-based homoserine lactone and bio-based organic acid, which are obtained from O-acyl homoserine produced by a microorganism through a chemical conversion process.

To achieve the above object, the present invention provides a method of producing homoserine lactone and organic acid through hydrolysis of O-acyl homoserine produced by a microorganism in the presence of hydrochloric acid, wherein O-acyl homoserine and hydrochloric acid are used at a molar ratio of 1:1-15.

The present invention also provides a method of producing gamma-butyrolactone by a hydrodenitrification or deamination of the said bio-based homoserine lactone.

The present invention also provides a method for producing tetrahydrofuran, 2-pyrrolidone, N-methyl-2-pyrrolidone, N-vinyl-2-pyrrolidone, 1,4-butanediol, etc., which are derivatives of the said gamma-butyrolactone.

The present invention also provides a method of producing ethanol, ethylene, polyethylene, monoethylene glycol, 1,4-butanediol, tetrahydrofuran, N-methyl-2-pyrrolidone, etc., using organic acids produced as byproducts together with bio-based homoserine lactone through hydrolysis of acyl homoserine produced by a microorganism in the presence of an acid catalyst.

### DETAILED DESCRIPTION OF THE INVENTION

A first embodiment of the present invention relates to a method of producing homoserine lactone and organic acid from microorganism-derived O-acyl homoserine by hydrolysis in the presence of hydrochloric acid, wherein O-acyl homoserine and hydrochloric acid are used at a molar ratio of 1:1-15.

As used herein, the term "O-acyl homoserine produced by a microorganism" refers to O-acyl homoserine produced by fermenting microorganisms.

The O-acyl homoserine includes O-acetyl-L-homoserine and O-succinyl-L-homoserine.

In the present invention, the microorganism may belong to any species that can be genetically engineered to produce O-acyl homoserine. Examples of microorganisms that may be used in the present invention include microorganisms of *Escherichia* sp., *Erwinia* sp., *Serratia* sp., *Providencia* sp., *Corynebacteria* sp., *Pseudomonas* sp., *Leptospira* sp., *Salmonellar* sp., *Brevibacteria* sp., *Hypomononas* sp., *Chromobacterium* sp. and *Norcardia* sp., fungi and yeasts. Preferably, the microorganism belongs to *Corynebacteria* sp. or *Escherichia* sp. More preferably, the microorganism is an *E. coli* strain that produces O-acyl homoserine. In addition, the microorganism is preferably a strain having enhanced O-acyl homoserine productivity by transformation.

The strain having enhanced O-acetyl-L-homoserine productivity is preferably a strain whose cystathionine gamma synthase activity, O-succinylhomoserine sulfhydrylase or O-acetylhomoserine sulfhydrylase activity was removed or weakened.

In addition, the strain having enhanced O-acyl homoserine productivity may be a strain having enhanced O-acetyl-L-homoserine productivity.

The strain having enhanced O-acetyl-L-homoserine productivity is preferably a strain whose homoserine O-acetyl transferase activity was enhanced.

Moreover, the strain having enhanced O-acyl homoserine productivity may be a strain having enhanced O-succinyl-L-homoserine productivity.

The strain having enhanced O-succinyl-L-homoserine productivity is preferably a strain whose homoserine O-succinyl transferase (MetA) activity was enhanced.

The present invention is characterized in that bio-based homoserine lactone is produced from the microorganism-derived O-acyl homoserine by hydrolysis in the presence of hydrochloric acid, wherein O-acyl homoserine and hydrochloric acid are used at a molar ratio of 1:1-15.

As used herein, the term "bio-based" means the material, for example, O-acyl homoserine is microbially produced and it is used to distinguish from a petrochemicals.

The acid catalyst is concentrated hydrochloric acid (35% or more; about 12M) or a dilute hydrochloric acid.

In the present invention, O-acyl homoserine and hydrochloric acid are used at a molar ratio of 1: 1-15.

The hydrolysis reaction is preferably performed either at 40~60 °C for 1-3 hours or under reflux for 1-3 hours.

The homoserine lactone produced by the method of the present invention may be deaminated to gamma-butyrolactone, which may then be used as a raw material for producing a variety of industrially highly useful materials, including tetrahydrofuran, 2-pyrrolidone, N-methyl-2-pyrrolidone, N-vinyl-2-pyrrolidone and the like.

Furthermore, the present invention is characterized in that bio-based organic acids are produced as byproducts together with the homoserine lactone.

The organic acids include acetic acid and succinic acid.

More specifically, the present invention is characterized in that, when O-acetyl-L-homoserine is used as O-acyl homoserine, acetic acid is produced as a byproduct together with the homoserine lactone, and when O-succinyl-L-homoserine is used as O-acyl homoserine, succinic acid is produced as a byproduct together with the homoserine lactone.

Acetic acid produced by the method of the present invention may be used as a raw material for producing a variety of industrially highly useful materials. It can be hydrogenated to ethanol according to a conventional method known in the art and the ethanol can be then dehydrated to ethylene, monoethylene glycol, ethyl acetate, diethyl ether, chloroform, iodoform, acetic acid, acetaldehyde, ethyl chloride, ethyl bromide, butadiene and the like. In addition, the ethylene can be polymerized to polymers such as polyethylene according to a method well known to those skilled in the art.

In addition, succinic acid produced by the method of the present invention can be hydrogenated to 1,4-butanediol in the presence of a catalyst, and the 1,4-butanediol may be used as a raw material for producing a variety of industrially highly useful materials and can be converted to gamma-butyrolactone, tetrahydrofuran and the like. In addition, succinic acid produced by the method of the present invention can be copolymerized with 1,4-butanediol to produce biodegradable polybutylene succinate.

The bio-based homoserine lactone produced as described above can be deaminated to gamma-butyrolactone, which may then be used as a raw material for producing a variety of industrially highly useful materials, including tetrahydrofuran, 2-pyrrolidone, N-methyl-2-pyrrolidone, N-vinyl-2-pyrrolidone, etc.

Another embodiment of the present invention relates to a method for producing gamma-butyrolactone, the method comprising the steps of: producing bio-based homoserine lactone and bio-based organic acid from microorganism-derived O-acyl homoserine by hydrolysis in the presence of hydrochloric acid, wherein O-acyl homoserine and hydrochloric acid are used at a molar ratio of 1:1-15; and deaminating the homoserine lactone by hydrodenitrification using a metal catalyst and hydrogen gas to yield gamma-butyrolactone.

The step of producing bio-based homoserine lactone and bio-based organic acid from microorganism-derived O-acyl homoserine is the same as theembodiment described above and can provide homoserine lactone by hydrolysis in the presence of an acid catalyst.

Then, the homoserine lactone can be converted to gamma-butyrolactone by hydrodenitrification using a metal catalyst and hydrogen gas. The metal catalyst used in the present invention may be a catalyst which at least one metal selected from palladium (Pd), platinum (Pt), nickel (Ni) and cobalt (Co) is supported on carbon (C) or silica. Herein, the hydrodenitrification reaction is preferably performed at a temperature of 100~500 °C and a hydrogen pressure of 10-100 bar.

After the reaction, the metal catalyst is recovered for use in a subsequent reaction, and the filtrate is concentrated and purified to yield gamma-butyrolactone.

The produced gamma-butyrolactone has a high boiling point of 204 °C. It not only can be used as an intermediate for synthesizing N-methyl-2-pyrrolidone, N-vinyl-2-pyrrolidone, polyvinyl pyrrolidone and the like, but also can be used as an intermediate for aromatic compounds, anti-rusting agents, secondary battery electrolytic solvents, medicines or agricultural chemicals which is an important material used in various fields, including agricultural, pharmaceutical, dye, pigments, fragrances, cosmetics, petrochemicals and electronic fields.

Another embodiment of the present invention relates to a method for producing tetrahydrofuran, the method comprising the steps of: producing bio-based homoserine lactone and bio-based organic acid from microorganism-derived O-acyl homoserine by hydrolysis in the presence of hydrochloric acid, wherein O-acyl homoserine and hydrochloric acid are used at a molar ratio of 1:1-15; deaminating the homoserine lactone by hydrodenitrification using a metal catalyst and hydrogen gas, thereby producing gamma-butyrolactone; and etherifying the gamma-butyrolactone with a silane compound in the presence of a indium bromide catalyst to yield tetrahydrofuran.

The process of producing gamma-butyrolactone from microorganism-derived O-acyl homoserine is the same as that of the embodiment described above.

The produced gamma-butyrolactone is dissolved in a solvent, and then etherified using a silane compound as a reducing agent in the presence of an indium bromide catalyst at 60~80 °C to yield tetrahydrofuran.

The solvent may be trichloromethane, benzene, toluene, acetonitrile or the like.

The silane compound is represented by the following formula 1:

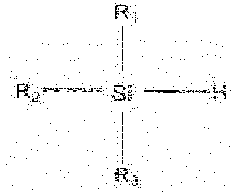

Wherein, R₁, R₂ and R₃ are selected from the same or different functional groups or atoms.

Preferred examples of the functional groups or atoms include a hydrogen atom, a halogen atom, an amino group, an alkyl group, a cycloalkyl group, an alkoxy group, a thioalkyl group, an alkylamino group, an aryl group, an arylamino group, a vinyl group, a siloxy group, an organo-silioxy group, an organo-silyl group, a heterocyclic group, and the like. The alkyl group, the cycloalkyl group, the alkoxy group, the thioalkyl group, the alkylamino group, the aryl group, the arylamino group, the vinyl group, the siloxy group, the organo-silioxy group, the organo-silyl group and the like generally have 1-18 carbon atoms, but are not limited thereto. In addition, R₁, R₂ and R₃ may also have a linear, branched-chain or cyclic structure, but at least one of R₁, R₂ and R₃ is preferably a C₁₋₄ alkyl group.

In addition, in formula 1, each of R₁, R₂ and R₃ is preferably the same or different R or XR (wherein, R is a C₁₋₄ alkyl group or an aryl group, and X is a heteroatom).

The indium bromide as a catalyst is preferably used in an amount of 2-100 mass%, and preferably 5-10 mass%, based on the amount of gamma-butyrolactone, and the silane compound is preferably used in an amount equivalent to 3-5 times, preferably 3.4-4.0 times that of the gamma-butyrolactone. Also, the indium bromide is used in an amount of 1-2 moles based on 100 moles of the silane compound.

The etherification reaction is preferably carried out at a temperature of 60~80 °C.

After completion of the reaction, the aqueous phase is extracted with dichloromethane (15 mL), dried over anhydrous Na₂SO₄, and evaporated under reduced pressure. The crude product is purified by flash column chromatography (SiO₂/hexane: AcOEt=99:1) to yield tetrahydrofuran.

Another embodiment of the present invention relates to a method for producing 2-pyrrolidone, the method comprising the steps of: producing bio-based homoserine lactone and bio-based organic acid from microorganism-derived O-acyl homoserine by hydrolysis in the presence of hydrochloric acid, wherein O-acyl homoserine and hydrochloric acid are used at a molar ratio of 1:1-15; deaminating the homoserine lactone by hydrodenitrification using a metal catalyst and hydrogen gas, thereby producing gamma-butyrolactone; and producing 2-pyrrolidone from the gamma-butyrolactone in the presence of an aqueous ammonia solution at high pressure and high temperature.

The process of producing gamma-butyrolactone from microorganism-derived O-acyl homoserine is the same as that of the embodiment described above.

The produced gamma-butyrolactone is mixed with an aqueous ammonia solution, and then reacted in a reactor with high pressure and high temperature at a temperature of 200~375 °C and a pressure of 40-100 bar for about 1-2 hours to produce 2-pyrrolidone.

The gamma-butyrolactone and ammonia are preferably used at a molar ratio of 1:0.5 to 1:1.5. If the molar ratio of gamma-butyrolactone used is higher than the upper limit of the above range, the production of 2-pyrrolidone will not increase, and other byproducts can be produced. For this reason, the amount of gamma-butyrolactone used is preferably within the above range.

The gamma-butyrolactone may be mixed with an aqueous ammonia solution in an anhydrous form or dissolved in water to prepare a gamma-butyrolactone solution before use.

The reaction temperature is preferably 200~375 °C. If the reaction temperature is lower than 200 °C, the reaction rate will be too low, and if the reaction temperature is higher than 375 °C, the concentration of byproducts other than 2-pyrrolidone will increase. For this reason, the reaction temperature is preferably within the above range.

The reaction pressure is preferably 40-100 bar, and the reaction time is preferably 10 minutes to 3 hours, and more preferably 1-2 hours.

Also, although 2-pyrrolidone can be produced by a batch process, it can be produced by a continuous process because it is preferable that the ammonia solution be progressively added during the process in order to reduce the production of byproducts from 4-hydroxy butyamide as an intermediate.

After completion of the reaction, water is removed, and the residue is extracted with chloroform. The resulting organic layer is dried with magnesium sulfate. Magnesium sulfate is removed by filtration, and then the filtrate is concentrated to yield 2-pyrrolidone.

Another embodiment of the present invention relates to a method for producing N-methyl-2-pyrrolidone, the method comprising the steps of: producing bio-based homoserine lactone and bio-based organic acid from microorganism-derived O-acyl homoserine by hydrolysis in the presence of hydrochloric acid, wherein O-acyl homoserine and hydrochloric acid are used at a molar ratio of 1:1-15; deaminating the homoserine lactone by hydrodenitrification using a metal catalyst and hydrogen gas, thereby producing gamma-butyrolactone; and producing N-methyl-2-pyrrolidone from the gamma-butyrolactone in the presence of liquid methylamine.

The process of producing gamma-butyrolactone from microorganism-derived O-acyl homoserine is the same as the method of the embodiment described above.

The produced gamma-butyrolactone is mixed with liquid methylamine, and the mixture is allowed to react at high temperature to yield N-methyl-2-pyrrolidone.

The gamma-butyrolactone and the methylamine are used at a molar ratio of 1:1-3 (gamma-butyrolactone: methylamine).

The reaction for producing N-methyl-2-pyrrolidone may be carried out in a microwave reactor, a Parr reactor, a reactor with high pressure and high temperature or the like.

The reaction conditions can vary depending on the reactor. When a microwave reactor is used, the reaction is preformed at a temperature of 180~220 °C at atmospheric pressure for 15 minutes to 1 hour, and preferably about 30 minutes, and when a Parr reactor is used, the reaction is performed at a temperature of 200~240 °C at a pressure of 10-20 bar for 3-5 hours, and preferably about 4 hours. when a reactor with high pressure and high temperature is used, the reaction is performed at a temperature of 250~300 °C at a pressure of 50 - 55 bar for 30 minutes to 2 hours, and preferably about 1 hour.

After completion of the reaction, water is removed, and the residue is extracted with chloroform. The resulting organic layer is dried with magnesium sulfate. Magnesium sulfate is removed by filtration, and the filtrate is concentrated to yield N-methyl-2-pyrrolidone.

Another embodiment of the present invention relates to a method for producing N-vinyl-2-pyrrolidone, the method comprising the steps of: producing bio-based homoserine lactone and bio-based organic acid from microorganism-derived O-acyl homoserine by hydrolysis in the presence of hydrochloric acid, wherein O-acyl homoserine and hydrochloric acid are used at a molar ratio of 1:1-15; deaminating the homoserine lactone by hydrodenitrification using a metal catalyst and hydrogen gas, thereby producing gamma-butyrolactone; dehydrating the gamma-butyrolactone in the presence of liquid ethyl alcohol amine to produce N-(2-hydroxyethyl)-2-pyrrolidone (first-stage reaction); and dehydrating the N-(2-hydroxyethyl)-2-pyrrolidone in the presence of an oxide catalyst containing an alkali metal or an alkaline earth metal and silicon to yield N-vinyl-2-pyrrolidone (second-stage reaction).

The process of producing gamma-butyrolactone from microorganism-derived O-acyl homoserine is the same as that of the embodiment described above.

The produced gamma-butyrolactone is dehydrated with ethyl alcohol amine in a liquid state to produce N-(2-hydroxyethyl)-2-pyrrolidone (first-stage reaction), which is then dehydrated in the presence of an oxide catalyst containing an alkali metal or an alkaline earth metal and silicon to yield N-vinyl-2-pyrrolidone (second-stage reaction).

More specifically, in the first-stage reaction, ethanol amine and water are introduced into an autoclave under a nitrogen atmosphere, and gamma-butyrolactone is added thereto with stirring, after which the autoclave is pressurized under 25-35 atm nitrogen pressure, and then the content in the autoclave is heated to about 200~250 °C and allowed to react for about 2 hours. In the first-stage reaction, a solution of N-(2-hydroxyethyl)-2-pyrrolidone is produced from the gamma-butyrolactone.

Then, the N-(2-hydroxyethyl)-2-pyrrolidone solution resulting from the first-stage reaction is distilled and purified to yield N-(2-hydroxyethyl)-2-pyrrolidone.

The second-stage reaction will now be described in detail.

First, cesium carbonate as the catalyst to be used in the second-stage reaction is dissolved in water, and silicon oxide is added thereto while the solution is heated to 90 °C with stirring. The mixture is heated, concentrated, and then dried in air at 120 °C for 20 hours. The resulting solid is crushed to a size of 9-16 mesh and calcined in air at 500 °C for 2 hours, thereby preparing a catalyst having a composition of Cs₁Si₁₀ (excluding oxygen).

Then, the catalyst is filled into a stainless reaction tube having an inner diameter of 15 mm, and the reaction tube is placed in a reactor under high temperature (about 360 °C). Then, feed gas which is N-(2-hydroxyethyl)-2-pyrrolidone diluted with nitrogen is supplied to the reactor at a space velocity of 200 hr⁻¹ and allowed to react at atmospheric pressure. After 1 hour from the initiation of the reaction, exit gas from the reactor is captured by methanol and purified by gas chromatography to yield N-vinyl-2-pyrrolidone.

The catalyst that is used in the second-stage reaction may be an oxide represented by the following formula 2:

Formula 2 MₐSi_{b}X_{c}O_{d}

Wherein, M is at least one element selected from alkali metals and alkaline earth metals, Si is silicon, X is at least one element selected from among B, Al and P, and O is oxygen. Also, if a is 1, b is 1-500, and c is 0-1, and d is determined by the values of a, b and c and the binding state of the elements.

The ratio of silicon to the alkali meal and/or the alkaline earth metal is depending on the kind of alkali meal and/or alkaline earth metal, but is typically 1-500: 1 (atomic ratio), and preferably 5-200: 1.

Also, X which is at least one element selected from among B, Al and P may be added optionally, the ratio of element X to the alkali meal and/or the alkaline earth metal is depending on the kind of alkali meal and/or alkaline earth metal, but is preferably 0-1: 1 (atomic ratio).

Another embodiment of the present invention relates to a method for producing 1,4-butanediol, the method comprising the steps of: producing bio-based homoserine lactone and bio-based organic acid from microorganism-derived O-acyl homoserine by hydrolysis in the presence of hydrochloric acid, wherein O-acyl homoserine and hydrochloric acid are used at a molar ratio of 1:1-15; deaminating the homoserine lactone by hydrodenitrification using a metal catalyst and hydrogen gas, thereby producing gamma-butyrolactone; and hydrogenating the gamma-butyrolactone to yield 1,4-butanediol.

The process of producing gamma-butyrolactone from microorganism-derived O-acyl homoserine is the same as that of the embodiment described above.

The produced gamma-butyrolactone is hydrogenated using 0.25 mol% of a ruthenium (Ru) as a catalyst and 1 mol% of an imadazole ligand in a THF solvent at 100 °C under a hydrogen gas pressure (50 bar) to produce 1,4-butanediol.

1,4-Butandiol has an annual worldwide market size of $4 billion and is used as a polymer intermediate and an industrial solvent. It is a raw material for producing polytetramethylene ether glycol that is a raw material for producing Spandex, and it reacts with a diisocyanate monomer to produce polyurethane resin. In addition, it is used for the production of polybutylene terephthalate that is a raw material for producing engineering plastic, and it may be used as an intermediate for the production of gamma-butyrolactone and the major solvent tetrahydrofuran.

Another embodiment of the present invention relates to a method for producing ethanol, the method comprising the steps of: producing bio-based homoserine lactone and bio-based acetic acid from microorganism-derived O-acetyl-L-homoserine by hydrolysis in the presence of hydrochloric acid, wherein O-acyl homoserine and hydrochloric acid are used at a molar ratio of 1:1-15; and hydrogenating the acetic acid in the presence of a catalyst comprising a first metal, a siliceous support and one or more support modifiers to produce ethanol.

The process of producing bio-based homoserine lactone and bio-based acetic acid from microorganism-derived O-acetyl-L-homoserine is the same as that of the embodiment described above.

Then, ethanol is produced from the acetic acid by hydrogenation in the presence of a catalyst.

The catalyst comprises a first metal, a siliceous support and one or more support modifiers.

The first metal may selected from the group consisting of platinum, copper, iron, cobalt, nickel, ruthenium, rhodium, palladium, osmium, iridium, titanium, zinc, chromium, rhenium, molybdenum and tungsten, and is preferably used in an amount of 0.1-25wt% based on the total weight of the catalyst.

The siliceous support may be selected from the group consisting of silica, silica alumina and calcium metasilicate and is preferably used in an amount of 25-99 wt% based on the total weight of the catalyst. Preferably, the surface area of the siliceous support is 50 m²/g-600 m²/g.

The support modifiers may be selected from the group consisting of oxides and metasilicates of sodium, potassium, magnesium, calcium, scandium, yttrium and zinc. Preferably, it is CaSiO₃ and is used in an amount of 0.1-50 wt% based on the total weight of the catalyst.

The catalyst may further comprise a second metal different from the first metal. The second metal may be selected from the group consisting of copper, molybdenum, tin, chromium, iron, cobalt, vanadium, tungsten, palladium, platinum, lanthanum, cerium, manganese, ruthenium, rhenium, gold and nickel. When the catalyst further comprises the second metal, the first metal and the second metal are preferably used in an amount of 0.1-10 wt% based on the total weight of the catalyst.

The hydrogenation is performed by passing hydrogen and acetic acid through the reactor at a gas hourly space velocity (GHSV) of 500 hr⁻¹ or more under a pressure of 10-3000 KPa at 125~350°C.

The ratio of hydrogen and acetic acid supplied is preferably more than 2:1.

In the presence of the above-described catalyst, ethanol is produced by the hydrogenation of acetic acid.

Ethanol produced as described above can be converted to ethylene according to a known method, for example, dehydration using concentrated sulfuric acid or gas-phase dehydration using activated alumina as a catalyst. In addition, ethanol can be converted to monoethylene glycol, ethyl acetate, diethyl ether, chloroform, iodoform, acetic acid, acetaldehyde, ethyl chloride, ethyl bromide, butadiene or the like. Further, ethylene can be polymerized to polymers such as polyethylene according to a well known polymerization method in the art.

Another embodiment of the present invention relates to a method for producing ethylene, the method comprising the steps of: producing bio-based homoserine lactone and bio-based acetic acid from microorganism-derived O-acetyl-L-homoserine by hydrolysis in the presence of hydrochloric acid, wherein O-acyl homoserine and hydrochloric acid are used at a molar ratio of 1:1-15; hydrogenating the acetic acid in the presence of a catalyst comprising a first metal, a siliceous support and one or more support modifiers to produce ethanol; and dehydrating the ethanol in the presence of a zeolite (ZSM-5) catalyst to produce ethylene.

The process of producing ethanol from microorganism-derived O-acetyl-L-homoserine is the same as that of the embodiment described above.

The produced ethanol is dehydrated in the presence of a catalyst to produce ethylene. The catalyst is preferably a zeolite (ZSM-5) catalyst.

In a preferred embodiment of the present invention, ethanol is placed in a fixed-bed quartz reactor and allowed to react at 550 °C to produce ethylene gas.

Another embodiment of the present invention relates to a method for producing polyethylene, the method comprising the steps of: producing bio-based homoserine lactone and bio-based acetic acid from microorganism-derived O-acetyl-L-homoserine by hydrolysis in the presence of hydrochloric acid, wherein O-acyl homoserine and hydrochloric acid are used at a molar ratio of 1:1-15; hydrogenating the acetic acid in the presence of a catalyst comprising a first metal, a siliceous support and one or more support modifiers to produce ethanol; and polymerizing the ethylene in the presence of a Ziegler-Natta catalyst to produce polyethylene.

The process of producing ethylene from O-acetyl-L-homoserine is the same as that of the embodiment described above.

The produced ethylene is polymerized in the presence of the Ziegler-Natta catalyst to produce polyethylene.

In a preferred embodiment of the present invention, the ethylene gas is polymerized in the presence of the Ziegler-Natta catalyst under a nitrogen gas pressure of 100 psi for 20 minutes at 50°C to produce polyethylene.

Another embodiment of the present invention relates to a method for producing monoethylene glycol, the method comprising the steps of: producing bio-based homoserine lactone and bio-based acetic acid from microorganism-derived O-acetyl-L-homoserine by hydrolysis in the presence of hydrochloric acid, wherein O-acyl homoserine and hydrochloric acid are used at a molar ratio of 1:1-15; hydrogenating the acetic acid in the presence of a catalyst comprising a first metal, a siliceous support and one or more support modifiers to produce ethanol; and producing monoethylene glycol from the ethanol in the presence of a Na₂PtCl₄ or Na₂PtCl₆ catalyst.

The process of producing ethanol from O-acetyl-L-homoserine is the same as that of the embodiment described above.

The produced ethanol is converted to monoethylene glycol in the presence of a catalyst.

The catalyst used is preferably a Na₂PtCl₄ or Na₂PtCl₆ catalyst.

In a preferred embodiment of the present invention, ethanol is reacted with the Na₂PtCl₄ or Na₂PtCl₆ catalyst to produce monoethylene glycol.

Another embodiment of the present invention relates to a method for producing 1,4-butanediol, the method comprising the steps of: producing bio-based homoserine lactone and bio-based succinic acid from microorganism-derived O-succinyl-L-homoserine by hydrolysis in the presence of hydrochloric acid, wherein O-acyl homoserine and hydrochloric acid are used at a molar ratio of 1:1-15; and hydrogenating the succinic acid in the presence of a metal catalyst on a carbon support to produce 1,4-butanediol.

The process of producing bio-based homoserine lactone and bio-based succinic acid from microorganism-derived O-succinyl-L-homoserine is the same as that of the embodiment described above.

The produced succinic acid is hydrogenated in the presence of a catalyst comprising palladium (Pd), silver (Ag) and rhenium (Re) metals on a carbon support to produce 1,4-butanediol.

The catalyst is prepared by impregnating a carbon support with a source of palladium (Pd) compound, a silver (Ag) compound and a rhenium (Re), drying the resulting carbon support at a temperature of 150 °C or below, removing a solvent from the impregnated carbon support, and heating the dried carbon support to a temperature of 100~350 °C under reducing conditions. The prepared catalyst includes crystalline palladium having an average particle size of 10 nm or less. At least one of sources of the palladium (Pd) compound, the silver (Ag) compound and the rhenium (Re) is a solution.

The carbon support preferably has a BET surface area of at least 200 m²/g, and preferably 500-1500 m²/g. The catalyst comprises 0.1-20 wt%, preferably 2-8 wt% of palladium (Pd), 0.1-20 wt%, preferably 1-8 wt% of silver (Ag) and 0.1-20 wt%, preferably 1-10 wt% of rhenium (Re). The ratio of palladium (Pd) to silver (Ag) is 10:1-1:10.

The palladium compound solution is a liquid solution containing a suitable amount of a palladium compound for a catalyst comprising a required amount of palladium. The palladium compound may be a palladium compound such as palladium nitrate or chloride, carbonate, carboxylate, acetate, acetyl acetonate or amine.

The silver compound solution is a liquid solution containing a suitable amount of a silver compound for producing a catalyst comprising a required amount of silver.

The palladium compound and the silver compound would have to be reduced into metals by thermal decomposition.

The rhenium compound solution is a liquid solution containing a suitable amount of a rhenium compound for producing a catalyst comprising a required amount of rhenium. The rhenium compound may be perrhenic acid, ammonium perrhenate or alkali metal perrhenate.

A method of contacting hydrogen or a hydrogen/nitrogen mixture with the catalyst can be used to reduce the catalyst.

In the presence of the catalyst prepared as described above, succinic acid is hydrogenated with a hydrogen-containing gas, and then purified by distillation to produce 1,4-butanediol.

The hydrogenation is carried out by contacting hydrogen and succinic acid at the ratio of 5:1-1000:1 for 0.1 minutes to 20 hours under a hydrogen pressure of 2-400 atm at 50~350°C.

The hydrogenation can provide, in addition to 1,4-butanediol, tetrahydrofuran, gamma-butyrolactone, n-butanol, n-butyric acid, n-propanol, and mixtures thereof, but the amounts of byproducts other than 1,4-butanediol and tetrahydrofuran are very insignificant.

The separation of 1,4-butanediol from the mixture can be performed by fractional distillation, and selectivity to 1,4-butanediol is up to 73.6%.

In the step of producing 1,4-butanediol from succinic acid in the presence of a catalyst comprising palladium (Pd), silver (Ag) and rhenium (Re) metals on a carbon support by hydrogenation, tetrahydrofuran can also be produced as a byproduct.

Another embodiment of the present invention relates to a method for producing gamma-butyrolactone, the method comprising the steps of: producing bio-based homoserine lactone and bio-based succinic acid from microorganism-derived O-succinyl-L-homoserine by hydrolysis in the presence of hydrochloric acid, wherein O-acyl homoserine and hydrochloric acid are used at a molar ratio of 1:1-15; hydrogenating the succinic acid in the presence of a metal catalyst(Pt, Pd or Ru) on a industrial MCM-41 support to produce gamma-butyrolactone and tetrahydrofuran.

The process of producing bio-based succinic acid from microorganism-derived O-succinyl-L-homoserine is the same as that of the embodiment described above.

The produced bio-based succinic acid is dehydrogenated in the presence of at least one catalyst selected from a group of Platinum, Palladium and Ruthenium, to produce gamma-butyrolactone and tetrahydrofuran.

### The catalyst

Another embodiment of the present invention relates to a method for producing gamma-butyrolactone, the method comprising the steps of: producing bio-based homoserine lactone and bio-based succinic acid from microorganism-derived O-succinyl-L-homoserine by hydrolysis in the presence of hydrochloric acid, wherein O-acyl homoserine and hydrochloric acid are used at a molar ratio of 1:1-15; hydrogenating the succinic acid in the presence of a metal catalyst on a carbon support to produce 1,4-butanediol; and dehydrogenating the 1,4-butanediol in the presence of a copper-zinc-based catalyst to produce gamma-butyrolactone.

The process of producing 1,4-butanediol from microorganism-derived O-succinyl-L-homoserine is the same as that of the embodiment described above.

The produced 1,4-butanediol is dehydrogenated in the presence of a copper-zinc-based catalyst to produce gamma-butyrolactone.

The copper-zinc-based catalyst is specifically Cu-ZnO-Al₂O₃-ZrO₃ produced by hydrogen reduction of plastic body (catalyst precursor) of precipitate obtained from a mixed solution of zinc nitrate, aluminum nitrate, zirconium nitrate and copper acetate and alkali hydroxide.

In the presence of the Cu-ZnO-Al₂O₃-ZrO₃ catalyst, 1,4-butanediol is dehydrogenated in a gas phase to produce gamma-butyrolactone.

The dehydrogenation is preferably performed at a temperature 150~400 °C at which 1,4-butanediol can be present in a gas phase. The dehydrogenation is performed in a reactor, which includes a ceramic ring-packed vaporization layer as an upper layer and a catalyst layer as a lower layer and has a carrier gas inlet and a raw material inlet at the top and a reaction solution capture container (cooling) having a gas outlet at the bottom, but is not limited thereto.

The yield of the gamma-butyrolactone produced by the above method is 97.9%.

**Anotherembodiment of the present invention relates to a method for producing tetrahydrofuran,**
the method comprising the steps of: producing bio-based homoserine lactone and bio-based succinic acid from microorganism-derived O-succinyl-L-homoserine by hydrolysis in the presence of hydrochloric acid, wherein O-acyl homoserine and hydrochloric acid are used at a molar ratio of 1:1-15; hydrogenating the succinic acid in the presence of a metal catalyst on a carbon support to produce 1,4-butanediol; and dehydrating the 1,4-butanediol in the presence of a catalyst selected from inorganic acid, tungstic oxide supported on alumina, and iron phosphate, to produce tetrahydrofuran.

The process of producing 1,4-butanediol from microorganism-derived O-succinyl-L-homoserine is the same as that of the embodiment described above.

The produced 1,4-butanediol is dehydrated in the presence of a catalyst selected from inorganic acid, tungstic oxide supported on alumina and iron phosphate, to produce tetrahydrofuran.

The inorganic acid catalyst is an acid catalyst such as sulfuric acid or cation exchange resin. When the organic acid catalyst is used, the production of tetrahydrofuran from 1,4-butanediol is performed by dehydrating 1,4-butanediol in a reaction column including a catalyst such as sulfuric acid or cation exchange resin under a pressure of 1-10 kg/cm² at 100~ 200 °C to obtain a reaction product including a mixture of water and tetrahydrofuran, introduing the reaction product into the extractive distillation column and extractive distillating continuously the reaction product under a pressure of 0.1-10 kg/cm² at 40~200°C using 1,4-butanediol as an extraction solvent.

In the liquid phase modification, the tungstic oxide catalyst supported on alumina may be prepared *in situ* by heating tungstic oxide, tungstic acid (H₂WO₄), or either of these substances compounds with a support such as alumina, silica, or the like in the presence of the 1,4-butanediol, optionally in a hydrogen atmosphere.

When the tungstic oxide catalyst is supported on alumina or silica on the like, a synergistic activating effect may be achieved. Thus, a catalyst prepared from a composition of 10% tungstic oxide and 90% aluminum oxide is substantially more active than one derived from tungstic oxide itself. When the tungstic oxide catalyst supported on alumina was used, the tube reactor was charged with 162 g (70 ml) of Harshaw tungsten catalyst WO 0801, 1/8 inch pellets containing 10% WO₃ and 90% Al₂O₃, and the bed was heated to 250°C under hydrogen flow at 70 ml per minute, and then 1,4-butanediol was passed into the boiler at 36 ml per hour. When a steady state was reached, tetrahydrofuran was obtained from the condensed effluent containing only tetrahydrofuran and water in 1:1 ratio.

The iron phosphate catalyst is prepared by adding phosphoric acid or ammonium phosphate to a 1M aqueous solution of iron nitrate at a P/Fe automic ratio of 1-1.5, stirring the mixture at 90°C for 2 hours, and drying the stirred mixture in a dryer for 24 hours. The iron phosphate catalyst may be used alone or with a support material such as alumina, silica, titania, zeolite or activated carbon. Preferably, the iron phosphate catalyst may be pretreated under flow of hydrogen or inert gas such as nitrogen, helium or argon at 200∼400 °C before use to increase the activity of the catalyst. When the iron phosphate catalyst was used, 1,4-butanediol and 1-20 wt% of iron phosphate catalyst based on the weight of 1,4-butanediol is loaded into a liquid phase reactor, and the tetrahydrofuran was obtained from the reactor by reacting at a temperature of 150~300 °C for about 1 hour.

Anotherembodiment of the present invention relates to a method for producing N-methyl-2-pyrrolidone, the method comprising the steps of: producing bio-based homoserine lactone and bio-based succinic acid from microorganism-derived O-succinyl-L-homoserine by hydrolysis in the presence of hydrochloric acid, wherein O-acyl homoserine and hydrochloric acid are used at a molar ratio of 1:1-15; hydrogenating the succinic acid in the presence of a metal catalyst on a carbon support to produce 1,4-butanediol; dehydrogenating the 1,4-butanediol in the presence of a copper-zinc-based catalyst to produce gamma-butyrolactone; and dehydrating the gamma-butyrolactone with liquid methylamine to produce N-methyl-2-pyrrolidone.

The process of producing gamma-butyrolactone from microorganism-derived O-succinyl-L-homoserine is the same as that of the embodiment described above.

The process of producing N-methyl-2-pyrrolidone from the produced gamma-butyrolactone comprises dehydrating the gamma-butyrolactone with liquid methylamine to produce N-methyl-2-pyrrolidone and allowing the mixture to react at high temperature.

Hereinafter, the present invention will be described in further detail with examples. It is to be understood, however, that these examples are for illustrative purposes only and are not intended to limit the scope of the present invention.

### Example 1: Construction of O-acyl homoserine producing strain

### 1-1) Deletion of metB gene

To deletion *metB* gene encoding cystathionine synthase in *E. coli* strain, FRT-one-step PCR deletion was performed (PNAS (2000) vol97: P6640-6645). Primers of SEQ. ID. NO: 1 and NO: 2 were used for PCR using pKD3 vector (PNAS (2000) vol97: P6640-6645) as a template, resulting in the construction of deletion cassette. PCR was performed as follows; 30 cycles of denaturation at 94°C for 30 seconds, annealing at 55°C for 30 seconds, extension at 72°C for 1 minute.

The PCR product was electrophoresed on 1.0% agarose gel, followed by purification of DNA obtained from 1.2 kbp band.

The recovered DNA fragment was electroporated into *E. coli* (K12) W3110 transformed with pKD46 vector (PNAS (2000) vol97: P6640-6645). Before electroporation, W3110 transformed with pKD46 was cultivated at 30°C in LB medium containing 100 µg/L of ampicilin and 5 mM of l-arabinose until OD₆₀₀ reached 0.6. Then, the cultured strain was washed twice with sterilized distilled water and one more time with 10% glycerol. Electroporation was performed at 2500 V. The recovered strain was streaked on LB plate medium containing 25 µg/L of chloramphenichol, followed by culture at 37°C for overnight. Then, a strain exhibiting resistance was selected.

PCR was performed by using the selected strain as a template with the same primers as the above under the same condition. The deletion of *metB* gene was identified by confirming the 1.2 kb sized gene on 1.0% agarose gel. The strain was then transformed with pCP20 vector (PNAS (2000) vol97: P6640-6645) and cultured in LB medium. The final *metB* knock-out strain was constructed in which the size of *metB* gene reduced to 150 bp on 1.0% agarose gel by PCR under the same conditions. Chloramphenicol marker was confirmed to be eliminated. The constructed strain was named W3-B.

### 1-2) Deletion of thrB gene

The inventors tried to increase O-succinylhomoserine synthesis from homoserine by deletion of *thrB* gene encoding homoserine kinase. To deletion *thrB* gene in the W3-B strain constructed above, FRT one step PCR deletion was performed by the same manner as described above for the deletion of *metB* gene.

To construct *thrB* deletion cassette, PCR was performed by using pKD4 vector (PNAS (2000) vol97: P6640-6645) as a template with primers of SEQ. ID. NO: 3 and NO: 4 under the same conditions as the above 1-1. The PCR product was electrophoresed on 1.0% agarose gel, followed by purification of DNA obtained from 1.6 kbp band.

The recovered DNA fragment was electroporated into the W3-B strain transformed with pKD46 vector. The recovered strain was streaked on LB plate medium containing 50 µg/L of kanamycin, followed by culture at 37°C for overnight. Then, a strain exhibiting resistance was selected.

PCR was performed by using the selected strain as a template with primers of SEQ. ID. NO: 3 and NO: 4 under the same conditions as the above. The deletion of *ThrB* gene was identified by selecting the strain whose size is 1.6 kb on 1.0% agarose gel. The strain was then transformed with pCP20 vector and cultured in LB medium. The final *thrB* knock out strain was constructed in which the size of *thrB* gene reduced to 150 kb on 1.0% agarose gel by PCR under the same conditions. Kanamycin marker was confirmed to be eliminated. The constructed strain was named W3-BT.

### 1-3) Deletion of metJ gene

To deletion *metJ* gene which is the regulator gene of *metA* gene involved in the O-acyl homoserine synthesis, FRT one step PCR deletion was performed by the same manner as used for the deletion of *metB* gene.

To construct *metJ* deletion cassette, PCR was performed with primers of SEQ. ID. NO: 5 and NO: 6 under the same conditions as the above 1-1.

The PCR product was electrophoresed on 1.0% agarose gel, followed by purification of DNA obtained from 1.2 kbp band. The recovered DNA fragment was electroporated into the W3-BT strain transformed with pKD46 vector. The recovered strain was streaked on LB plate medium containing chloramphenicol, followed by culture at 37°C for overnight. Then, a strain exhibiting resistance was selected.

PCR was performed by using the selected strain as a template with primers of SEQ. ID. NO: 7 and NO: 8 under the same conditions as the above. The deletion of *metJ* was identified by confirming the 1.6kb sized gene on the 1.0% agarose gel. The strain was then transformed with pCP20 vector and cultured in LB medium. The final *metJ* knock out strain was constructed in which the size of *metJ* gene reduced to 600 kb on 1.0% agarose gel by PCR under the same conditions and the strain Chloramphenicol marker was confirmed to be eliminated. The constructed strain was named W3-BTJ.

### 1-4-1) Over-expression of metA gene

To increase O-acylhomoseine synthesis, *metA* gene encoding homoserine O-succinyl transferase involved in the synthesis of O-succinyl homoserine from homoserine, was over-expressed.

PCR was performed by using the chromosome of *E. coli* w3110 as a template with primers of SEQ. ID. NO: 9 and NO: 10 as follows; 25 cycles of denaturation at 94°C for 30 seconds, annealing at 55°C for 30 seconds, extension at 72°C for 2 minutes.

The PCR product was electrophoresed on 1.0% agarose gel, followed by purification of DNA obtained from 1.2 kbp band. The recovered DNA fragment was ligated to another DNA fragment obtained from pCL1920 vector by digesting with *Sma*I. *E. coli* was transformed with the ligated vector, which was then cultured in LB medium containing 50 µg/L of spectinomycin, followed by selection. The vector constructed thereby was named pMetA-CL. W3-BTJ strain was transformed with the said vector. The constructed strain was named W3-BTJ/pMetA-CL and the increase of O-succinylhomoserine level therein was observed.

As another method to increase *metA* gene expression, *metA* gene was ligated to pCL1920 vector by using CJ1 promoter (CJ, Korea, Korean Patent Registration No. 10-0620092) and *E. coli* was transformed with the ligated vector, which was then cultured in LB medium containing 50 µg/L of spectinomycin, followed by selection. The vector constructed thereby was named pCJ-MetA-CL. W3-BTJ strain was transformed with the said vector. The constructed strain was named W3-BTJ/pCJ-MetA-CL and the increase of O-succinylhomoserine level therein was observed.

### 1-4-2) Over-expression of metX gene

To synthesize O-acetylhomoserine, *metX* gene encoding homoserine O-acetyl transferase involved in the synthesis of O-acetylhomoserine from homoserine, was over-expressed.

PCR was performed by using the chromosome of *Leptospira meyeri* as a template with primers of SEQ. ID. NO: 11 and NO: 12 as follows under the same conditions as the above 1-4-1.

The PCR product was electrophoresed on 1.0% agarose gel, followed by purification of DNA obtained from 1.1 kbp band. The recovered DNA fragment was ligated to pCL1920 vector by using CJ1 promoter. *E. coli* was transformed with the ligated vector, which was then cultured in LB medium containing 50 µg/L of spectinomycin, followed by selection. The vector constructed thereby was named pCJ1-MetXlme-CL. W3-BTJ strain was transformed with the said vector. The constructed strain was named W3-BTJ/pCJ-MetXlme-CL and the increase of O-acetylhomoserine level therein was observed.

Another method to over-express *metX* gene was made by performing PCR using the chromosome of *Corynebacterium* as a template with primers of SEQ. ID. NO: 13 and NO: 14 under the same conditions as the above 1-4-1..

The PCR product was electrophoresed on 1.0% agarose gel, followed by purification of DNA. The recovered DNA fragment was ligated to pCL1920 vector by using CJ1 promoter. *E. coli* was transformed with the ligated vector, which was then cultured in LB medium containing 50 µg/L of spectinomycin, followed by selection. The vector constructed thereby was named pCJ-MetXcgl-CL. W3-BTJ strain was transformed with the said vector. The constructed strain was named W3-BTJ/pCJ-MetXcgl-CL and the increase of O-acetylhomoserine level therein was observed.

### 1-4-3) Deletion of metA gene

To increase the production of O-acetylhomoserine, *metA* gene encoding homoserine O-succinyl transferase was deleted in W3-BTJ strain. Based on the founding that only *metX* gene introduction resulted in the accumulation of O-succinylhomoserine, it was expected that *metA* gene deletion resulted in the promotion of the accumulation of O-acetylhomoserine (see, Table 3). To deletion *metA* gene, FRT one step PCR deletion was performed.

To construct *metA* deletion cassette, PCR was performed with primers of SEQ. ID. NO: 15 and NO: 16 under the same conditions as the above 1-1.

The PCR product was electrophoresed on 1.0% agarose gel, followed by purification of DNA obtained from 1.2 kbp band. The recovered DNA fragment was electroporated into the *E. coli* W3-BTJ strain transformed with pKD46 vector. The recovered strain was streaked on LB plate medium containing chloramphenicol, followed by culture at 37°C for overnight. Then, a strain exhibiting resistance was selected.

PCR was performed by using the selected strain as a template with primers of SEQ. ID. NO: 15 and NO: 16 under the same conditions as the above. The deletion of *metA* gene was identified by confirming 1.1 kb sized gene on 1.0% agarose gel. The strain was then transformed with pCP20 vector and cultured in LB medium. The final *metA* knock out strain was constructed in which the size of *metA* gene reduced to 100 kb on 1.0% agarose gel by PCR under the same conditions. Chloramphenicol marker was confirmed to be eliminated. The constructed strain was named W3-BTJA. The W3-BTJA strain was transformed with the pCJ-MeTXlme-CL vector and the resultant strain was named W3-BTJA/pCJ-MetX-CL. The strain was cultured by the same manner as described above and as a result the accumulation of O-succinylhomoserine was not observed, but the production of O-acetylhomoserine was significantly, approximately 20% increased, compared with W3-BTJ.

### 1-5) Transformation of L-threonine producing strain

O-acyl homoserine producing strains were constructed by the same manner as described in Examples <1-1> to <1-3> using *E. coli* CJM002 (KCCM-10568), the L-threonine producing strain free from the requirement for methionine. The constructed strains were named CJM-BTJ.

In addition, CJM-BTJ/pMetA-CL (accession number: KCCM-10767) and CJM-BTJ/pCJ-MetA-CL (accession number: KCCM-10872) were constructed in the same manner as Example 1-4-1. The CJMBTJ/pMetA-CL and CJM-BTJ (pCJ-MetA-CL) strains were O-succinyl homoserine-producing *E. coli* strains transformed so as to have deletions of *metB*, *thrB* and *metJ* and over-express *metA.* However, the CJM-BTJ(pCJ-MetA-CL) strain was constructed using the CJ1 promoter in order to over-express *metA,* unlike the CJM-BTJ/pMetA-CL strain (accession number: KCCM-10767).

The *metX* gene overexpressed, *metA* gene knock-out strain was also constructed by the same manner as described in <1-4-2> and <1-4-3> using the CJM-BTJ strain and the resultant strain was named CJM-BTJA (pCJMetX-CL) (accession number: KCCM-10873). This strain is an *E. coli* strain transformed so as to have deletions of *metB*, *thrB*, *metJ* and *metA* and overexpress *metX*, and it has improved ability to produce O-acetyl-L-homoserine.

### Example 2: Fermentation for the production of O-acyl homoserine

To investigate the O-acyl homoserine production capacity of the strain constructed in Example 1, Erlenmeyer flask culture was performed. The compositions of production medium are shown in Table 1 below.

W3-BTJ, CJM-BTJ and W3-BTJ and CJM-BTJ transformed with *metA* and *metX* expression vector were cultured on LB plate media containing spectinomycin at 31°C for overnight. A single colony was inoculated in 3 ml of LB medium containing spectinomycin, followed by culture at 31°C for 5 hours. The culture solution was 200 fold diluted in 250 ml Erlenmeyer flask containing 25 ml of methionine precursor producing medium, followed by culture at 31°C, 200 rpm for 64 hours. HPLC was performed to compare with O-acyl homoserine production capacity (see, Table 2 and Table 3).

As a result, O-acyl homoserine capacity was significantly increased in the producing strain prepared from the L-threonine producing strain free from the requirement for methionine.

**Table 1: Flask medium compositions for O-acyl homoserine production**

| Composition | Concentration (per liter) |
|---|---|
| Glucose | 40 g |
| Ammonium sulfate | 17 g |
| KH₂PO₄ | 1.0 g |
| MgSO₄ · 7H₂O | 0.5 g |
| FeSO₄ · 7H₂O | 5 mg |
| MnSO₄ · 8H₂O | 5 mg |
| ZnSO₄ | 5 mg |
| Calcium carbonate | 30 g |
| Yeast extract | 2 g |
| Methionine | 0.15 g |
| Threonine | 0.15 g |

**Table 2: O-succinyl-L-homoserine production by flask culture**

| Strain | OD | Glucose consumption (g/L) | O-succinyl-L-homoserine (g/L) |
|---|---|---|---|
| W3-BTJ | 10 | 40 | 0.3 |
| W3-BTJ/ pMetA-CL | 12 | 40 | 1.2 |
| W3-BTJ/ pCJ-MetA-CL | 12 | 40 | 1.8 |
| CJM-BTJ | 5.0 | 33 | 0.6 |
| CJM-BTJ/ pMetA-CL (KCCM-10767) | 6.0 | 36 | 5.2 |
| CJM-BTJ/pCJ-MetA-CL (KCCM-10872) | 6.0 | 40 | 10.1 |

**Table 3: O-acetyl-L-homoserine production by flask culture**

| | OD | Glucose consumption (g/L) | O-acetyl-L-homoserine (g/L) |
|---|---|---|---|
| W3-BTJ | 10 | 40 | 0 |
| W3-BTJ/pCJ-MetXlme-CL | 12 | 40 | 1.5 |
| W3-BTJ/pCJ-MetXcgl-CL | 12 | 40 | 1.4 |
| W3-BTJA/pCJ-MetXlme | 11 | 40 | 1.8 |
| CJM-BTJ | 5.0 | 33 | 0 |
| CJM-BTJ/pCJ-MetXlme-CL | 5.5 | 40 | 4.8 |
| CJM-BTJ/pCJ-MetXcgl-CL | 6.0 | 36 | 4.6 |
| CJM-BTJA/pCJ-MetX-CL (KCCM-10873) | 5.8 | 40 | 6.5 |

For mass production of O-acyl homoserine, 5-L fermentor culture was performed. The compositions of medium used in the fermentor are shown in Table 4 below.

CJM-BTJ/pCJ-metA-CL (accession number: KCCM-10872) or CJM-BTJA/pCJ-metX-CL (accession number: KCCM-10873) was inoculated in LB medium containing spectinomycin, followed by culture at 31°C for overnight.

Then, a single colony was inoculated in 10 ml LB medium containing spectinomycin, which was cultured at 31°C for 5 hours. The culture solution was 100 fold diluted in 1000 ml Erlenmeyer flask containing 200 ml of O-acyl homoserine seed medium, followed by culture at 31°C, 200 rpm for 3 - 10 hours. The culture solution was inoculated in a 5 L fermentor, followed by further culture for 50 - 100 hours by fed-batch fermentation. The O-acyl homoserine concentration in the fermented solution was measured by HPLC and the results are shown in Table 5.

**Table 4: Fermentor medium compositions for O-acyl homoserine production**

| Composition | Seed medium | Main medium | Feed medium |
|---|---|---|---|
| Glucose (g/L) | 10.1 | 40 | 600 |
| MgSO₄ · 7H₂O (g/L) | 0.5 | 4.2 | |
| Yeast extract (g/L) | 10 | 3.2 | |
| KH₂PO₄ | 3 | 3 | 8 |
| Ammonium sulfate (g/L) | | 6.3 | |
| NH₄Cl (g/L) | 1 | | |
| NaCl (g/L) | 0.5 | | |
| Na₂HPO4 · 12H₂O (g/L) | 5.07 | | |
| DL-methionine (g/L) | | 0.5 | 0.5 |
| L-isoleucine (g/L) | 0.05 | 0.5 | 0.5 |
| L-threonine (g/L) | | 0.5 | 0.5 |

**Table 5: O-acyl homoserine production in a fermentor**

| Strain | O-succinyl-L-homoserine (g/L) | O-acetyl-L-homoserine (g/L) |
|---|---|---|
| CJM-BTJ/pCJ-MetA-CL (KCCM-10872) | >80 | 0 |
| CJM-BTJA/pCJ-MetX-CL (KCCM-10873) | 0 | >55 |

### Example 3: Synthesis of homoserine lactone and organic acid from O-acyl homoserine

The following Examples were performed using O-acyl homoserines, especially O-acetyl-L-homoserine and O-succinyl-L-homoserine, produced by microorganisms in Example 2.

### 3-1) Synthesis of homoserine lactone and acetic acid from O-acetyl-L-homoserine

(1) 2 g (12.4 mmol) of O-acetyl-L-homoserine was completely dissolved in 10 ml (120 mmol, 9.7 equivalents) of concentrated hydrochloric acid, and the solution was allowed to react at 50°C for 2 hours, followed by removal of the hydrochloric acid, thereby obtaining 1.7 g (12.3 mmol) of homoserine lactone hydrochloride (purity: 99%).
   ¹H NMR (300 MHz, DMSO) δ 8.83 (2H, brs), 4.46 (1H, t, *J =* 8.8 Hz), 4.36-4.24 (2H, *m*), 2.61-2.51 (1H, *m*), 2.30 (1H, t, *J* = 10.3 Hz)
   ¹H NMR (300 MHz, D₂O) δ 4.36 (1H, t, *J* = 9.0 Hz), 4.29 (2H, *q*, *J* = 9.0 Hz), 2.69-2.60 (1H, *m*), 2.36-2.21 (1H, *m*) 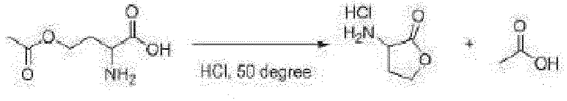
(2) 2 g (12.4 mmol) of O-acetyl-L-homoserine was completely dissolved in a mixture of 1.13 ml (13.6 mmol, 9.7 equivalents) (1.24 M) of concentrated hydrochloric acid and 10 ml of water, and the solution was allowed to react under reflux for 2 hours, followed by removal of the hydrochloric acid, thereby obtaining 1.7 g (12.3 mmol) of homoserine lactone chloride (purity: 99%).
   ¹H NMR (300 MHz, DMSO) δ 8.83 (2H, *brs*), 4.46 (1H, t, *J =* 8.8 Hz), 4.36-4.24 (2H, *m*), 2.61-2.51 (1H, *m*)*,* 2.30 (1H, t, *J* = 10.3 Hz)
   ¹H NMR (300 MHz, D₂O) δ 4.36 (1H, t, *J* = 9.0 Hz), 4.29 (2H, *q*, *J* = 9.0 Hz), 2.69-2.60 (1H, *m*), 2.36-2.21 (1H, *m*) 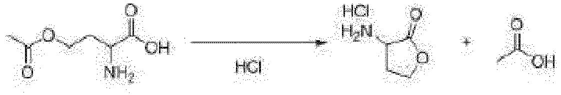
(3) 10 g (62.1 mmol) of O-acetyl-L-homoserine was completely dissolved in 50 ml (1.24 M) of water and 5.7 ml (68.3 mmol, 1.1 equivalents) of concentrated hydrochloric acid, and the solution was allowed to react under reflux for 2 hours, followed by removal of the solvent, thereby obtaining 8.5 g (61.8 mmol) of homoserine lactone hydrochloride (purity: 99%).
   ¹H NMR (300 MHz, DMSO) δ 8.83 (2H, *brs*), 4.46 (1H, *t*, *J =* 8.8 Hz), 4.36-4.24 (2H, *m*), 2.61-2.51 (1H, *m*), 2.30 (1H, *t*, *J* = 10.3 Hz)
   ¹H NMR (300 MHz, D₂O) δ 4.36 (1H, *t*, *J* = 9.0 Hz), 4.29 (2H, *q*, *J* = 9.0 Hz), 2.69-2.60 (1H, *m*), 2.36-2.21 (1H, *m)*

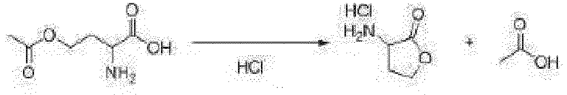

### 3-2) Synthesis of homoserine lactone and succinic acid from O-succinyl-L-homoserine

2 g (9.12 mmol) of O-succinyl-L-homoserine was dissolved in 10 ml (120 mmol, 13.2 equivalents) of concentrated hydrochloric acid, and the solution was allowed to react at 50°C for 2 hours, and then cooled at room temperature for 3 hours. The precipitated solid was filtered, thereby obtaining 0.7 g (5.9 mmol) of succinic acid (SA) crystal (purity: 65%). The filtrate was concentrated and recrystallized from anhydrous ethanol to yield 1.2 g (8.72 mmol) of homoserine lactone hydrochloride (purity: 95%).

¹H NMR (300 MHz, DMSO) δ 8.83 (2H, *brs*), 4.46 (1H, *t*, *J =* 8.8 Hz), 4.36-4.24 (2H, *m*), 2.61-2.51 (1H, *m*), 2.30 (1H, *t*, *J* = 10.3 Hz): Homoserine lactone hydrochloride

¹H NMR (300 MHz, D₂O) δ 4.36 (1H, *t*, *J* = 9.0 Hz), 4.29 (2H, *q*, *J* = 9.0 Hz), 2.69-2.60 (1H, *m*), 2.36-2.21 (1H, *m*): Homoserine lactone hydrochloride

¹H NMR (300 MHz, D₂O) δ 2.47 (4H, s): Succinic acid

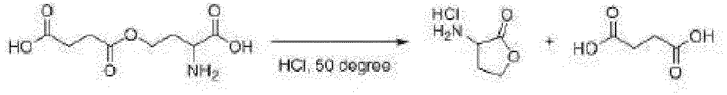

### Example 4: Synthesis of gamma-butyrolactone from homoserine lactone

The homoserine lactone hydrochloride obtained in Example 3 was loaded in a reactor and hydrodenitrified with hydrogen gas under a pressure of 10-100 bar at 100~500°C in the presence of a catalyst which supported on Pd, Pt, Ni or Co on C or silica, to produce gamma-butyrolactone.

### Example 5: Synthesis of tetrahydrofuran from gamma-butyrolactone

The gamma-butyrolactone obtained on Example D was dissolved in a solvent, and then etherified using a silane compound as a reducing agent at 60~80 °C in the presence of an indium bromide catalyst to produce tetrahydrofuran.

The ¹H NMR spectrum was measured at 500 MHz using tetramethylsilane as an internal standard. The NMR spectrum was measured at 125 MHz using the central peak of chloroform (77.0 ppm) as an internal standard. High-resolution mass spectrometry was performed using NBA (3-nitrobenzylalcohol) as a matrix.

Under nitrogen atmosphere, gamma-butyrolactone (0.6 mmol), InBr₃ (10.6 mg, 0.0300 mmol) and triethylsilane (380 µL, 2.40 mmol) were sequentially added to 0.6 mL of distilled chloroform solution included in a vial with a screw cap, and the vial was sealed with a cap having a PTFE film. When the reaction continued to stir at 60 °C, the colorless solution changed to orange via yellow. The reaction was monitored by gas chromatography until the starting material gamma-butyrolactone was consumed. After completion of the reaction, water (3 mL) was added to the reaction product, and the orange suspension was continuously stirred until it became colorless. The aqueous phase was extracted with dichloromethane (15 mL), dried over anhydrous Na₂SO₄ and evaporated under reduced pressure. The crude product was purified by flash column chromatography (SiO₂/hexane: AcOEt=99:1) to yield tetrahydrofuran.

### Example 6: Synthesis of 2-pyrrolidone from gamma-butyrolactone

Using the gamma-butyrolactone obtained in Example 4, 2-pyrrolidone was synthesized in an autoclave.

More specifically, 6.45 g (75 mmol) of gamma-butyrolactone and 10.9 g (1.1 equivalents, 82.5 mmol, 12 ml) of NH₄OH (26.5% in water) were placed in an autoclave, and 250 ml (0.3 M) of water was added thereto. The solution was allowed to react under a pressure of 53 bar at 270 °C for 1 hour.

After completion of the reaction, the starting material gamma-butyrolactone was not observed on TLC, and new spots were produced.

Water was removed from the reaction product, and the residue was extracted with CHCl₃. The organic layer was dried with MgSO₄. MgSO₄ was filtered out, and the filtrate was concentrated and analyzed by NMR. As a result, it could be seen that 6 g (70.5 mmol, 94%) of 2-pyrrolidone was produced.

¹H NMR (300 MHz, CDCl₃) δ 6.61 (1H, *brs*), 3.39 (2H, *t*, *J* = 4.2 Hz), 2.28 (2H, *t*, *J* = 5.6 Hz), 2.15~2.02 (2H, *m)*

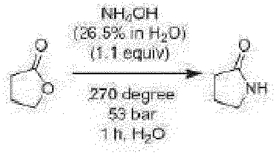

### Example 7: Synthesis of N-methyl-2-pyrrolidone from gamma-butyrolactone

Using the gamma-butyrolactone obtained in Example 4, N-methyl-2-pyrrolidone was synthesized under various reaction conditions as follows.

### 7-1) Synthesis of N-methyl-2-pyrrolidone from gamma-butyrolactone using microwave reactor

N-methyl-2-pyrrolidone can be obtained by allowing gamma-butyrolactone and methylamine to react with each other in a water solvent at high temperature in a microwave reactor.

0.2 g (2.23 mmol) of gamma-butyrolactone and 0.36 g (2.0 equivalents, 4.64 mmol) of methylamine (40% in water) were placed in a 5 ml microwave reactor, and 5 ml (0.46 M) of water was added thereto. Then, the solution was allowed to react at 200°C for 30 minutes. After completion of the invention, the starting material gamma-butyrolactone was not observed on TLC, and new spots were produced. NMR analysis of the crude product showed that N-methyl-2-pyrrolidone was produced at a yield of 80%.

¹H NMR (300 MHz, CDCl₃) δ 3.32 (2H, *t*, *J* = 5.3 Hz), 2.77 (3H, *s*), 2.30 (2H, *t*, *J* = 6.2 Hz), 1.99-1.91 (2H, *m*)

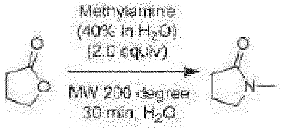

### 7-2) Synthesis A of N-methyl-2-pyrrolidone from gamma-butyrolactone using Parr reactor

3.18 g (36.9 mmol) of gamma-butyrolactone and 6.44 g (2.0 equiv, 73.88 mmol, 7.23 ml) of methylamine (40% in water) were placed in a Parr reactor, and 100 ml (0.37 M) of water was added thereto. Then, the solution was allowed to react in the Parr reactor under a pressure of 15 bar at 220 °C for 4 hours.

After completion of the invention, the starting material gamma-butyrolactone was not observed on TLC, and new spots were produced. NMR analysis of the crude product showed that N-methyl-2-pyrrolidone was produced at a yield of 50%.

¹H NMR (300 MHz, CDCl₃) δ 3.32 (2H, *t*, *J* = 5.3 Hz), 2.77 (3H, *s*), 2.30 (2H, *t*, *J* = 6.2 Hz), 1.99-1.91 (2H, *m)*

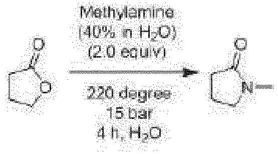

### 7-3) Synthesis B of N-methyl-2-pyrrolidone from gamma-butyrolactone using Parr reactor

3.22 g (37.4 mmol) of gamma-butyrolactone and 2.9 g (1.0 equiv, 37.4 mmol, 3.3 ml) of methylamine (40% in water) were placed in a Parr reactor, and 100 ml (0.37 M) of water was added thereto. Then, the solution was allowed to react in the Parr reactor under a pressure of 15 bar at 220 °C for 4 hours.

After completion of the invention, the starting material gamma-butyrolactone (GBL) was not observed on TLC, and new spots were produced. NMR analysis of the crude product showed that N-methyl-2-pyrrolidone was produced at a yield of 60%.

¹H NMR (300 MHz, CDCl₃) δ3 .32 (2H, *t*, *J* = 5.3 Hz), 2.77 (3H, *s*), 2.30 (2H, *t*, *J* = 6.2 Hz), 1.99-1.91 (2H, *m*)

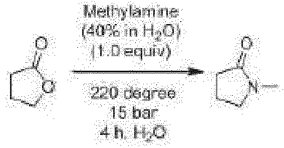

### 7-4) Synthesis of N-methyl-2-pyrrolidone from gamma-butyrolactone using autoclave

6.45 g (75 mmol) of gamma-butyrolactone and 6.4 g (1.1 equiv, 82.5 mmol, 7.1 ml) of methylamine (40% in water) were placed in an autoclave, and 250 ml (0.3 M) of water was added thereto. Then, the solution was allowed to react under a pressure of 53.3 bar at 270 °C for 1 hour. After completion of the invention, the starting material gamma-butyrolactone (GBL) was not observed on TLC, and new spots were produced. Water was removed from the reaction product, and the residue was extracted with CHCl₃. The organic layer was dried with MgSO₄. MgSO₄ was filtered out, and the filtrate was concentrated and analyzed by NMR. As a result, it could be seen that 6.92 g (69.8 mmol, 93%) of N-methyl-2-pyrrolidone was produced.

¹H NMR (300 MHz, CDCL₃) δ 3.32 (2H, t, *J* = 5.3 Hz), 2.77 (3H, *s),* 2.30 (2H, t, J = 6.2 Hz), 1.99-1.91 (2H, *m*)

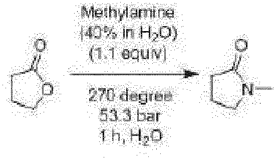

### Example H: Preparation of N-vinyl-2-pyrrolidone from gamma-butyrolactone

Using the gamma-butyrolactone obtained in Example D, N-vinyl-2-pyrrolidone was produced by a first-stage reaction and a second stage reaction.

### 8-1) First-stage reaction: Production of N-(2-hydroxyethyl)-2-pyrrolidone from gamma-butyrolactone

356 g of ethanolamine and 100 g of water were placed in a 1-liter autoclave, which is kept under nitrogen atmosphere, at room temperature and 518 g of gamma-butyrolactone was added thereto with stirring. Then, the inside of the autoclave was pressurized with a 30 atm of nitrogen atmosphere and heated to 250°C, and the mixture was allowed to react for 2 hours.

Then, the reaction solution was cooled and analyzed by gas chromatography. The analysis showed that the yield of N-(2-hydroxyethyl)-2-pyrrolidone was 94 mol%.

The reaction solution was purified by distillation to yield N-(2-hydroxyethyl)-2-pyrrolidone.

### 8-2) Second-stage reaction: Production of N-vinyl-2-pyrrolidone from N-(2-hydroethyl)-2-pyrrolidone

To prepare as a catalyst to be used in the second-stage reaction, 7.76 g of cesium carbonate was dissolved in 250 g of water, and 30 g of silicon oxide was added thereto while the solution was heated to 90°C and stirred. The mixture was dried at 120°C for 20 hours. The resulting solid was crushed to size of 9-16 mesh, calcined in air at 500°C for 2 hours, thereby preparing a catalyst having a composition of Cs₁Si₁₀ (expressed as atomic ratio excluding oxygen).

30 ml of the catalyst was charged into a stainless steel reaction tube having an inner diameter of 15 mm, and the reaction tube was placed in a reactor at 360 °C. Raw material gas obtained by diluting N-(2-hydroxyethyl)-2-pyrrolidone in nitrogen to reach a partial pressure of 76 mmHg was supplied to the reaction tube at a space velocity of 200 h⁻¹ and allowed to react at atmospheric pressure. After 1 hour from the initiation of the reaction, gas discharged from the reactor was captured by methanol, and the gas chromatography analysis of the gas showed that the yield of N-vinyl-2-pyrrolidone was 87 mole%.

### Example 9: Production of 1,4-butanediol from gamma-butyrolactone

The gamma-butyrolactone obtained in Example 4 was hydrogenated with hydrogen gas (50 bar) using 0.25 mol% of a ruthenium (Ru) catalyst and 1 mol% of an imidazole ligand in a THF solvent at 100 °C to yield 1,4-butanediol (Chem. Eur. J. 2012. 18, 9011-9018).

### Example 10: Production of ethanol from acetic acid

Acetic acid produced as a byproduct in Example 3-1 was hydrogenated in the presence of a catalyst comprising a first metal, a second metal, a siliceous support and at least one support modifier to yield ethanol.

The catalyst used was a SiO₂-CaSiO₃ -Pt-Sn catalyst prepared using Pt and Sn as the first and second metals, SiO₂ as the support and CaSiO₂ as the support modifier.

The hydrogenation reaction was performed by supplying hydrogen and acetic acid to the reactor at a pressure of 100 KPa at 250°C and a gas hourly space velocity (GHSV) of 500 hr⁻¹ or higher. The molar ratio of hydrogen and acetic acid supplied was 11:1.

The hydrogenation reaction yielded 600 g or more of ethanol per kg of the catalyst.

### Example 11: Production of ethylene from ethanol

The ethanol obtained in Example 10 was allowed to react at 550 °C in the presence of a zeolite (ZSM-5) catalyst in a fixed-bed quartz reactor to produce ethylene (Catalysis, A: General, 2012, 162-167).

### Example 12: Production of polyethylene from ethylene

Ethylene gas obtained in Example 11 was allowed to react under nitrogen atmosphere (100 psi) at 50°C for 20 minutes in the presence of a Ziegler-Natta catalyst to produce polyethylene (GB patent 1,406,282; 27 Jan 1972).

### Example 13: Production of monoethylene glycol from ethanol

The ethanol obtained in Example 10 was allowed to react with a Na₂PtCl₄ or Na₂PtCl₆ catalyst to produce monoethylene glycol (J. Am. Chem. Soc., 1994, 116, 998-1003).

### Example 14: Production of 1,4-butanediol from succinic acid

The succinic acid produced in Example 3-2 was hydrogenated in the presence of a catalyst comprising palladium, silver and rhenium metals on a carbon support to produce 1,4-butanediol.

### N-1): Preparation of catalyst

The catalyst to be used in hydrogenation was prepared in the following manner.

130.25 g of palladium nitrate solution (7.7% Pd), 16.5 g of silver nitrate and 41.5 g of perrhenic acid (52.6%, Re) were placed in a 250-cc flask, and acetonitrile was added thereto. The mixture was stirred to form a solution. The solution had a weight of 296.2 g.

Then, 276.5 g of 1.5 mm ACL40 (manufactured by CECA S.A. (France; Marketed by Atochem North America Inc.) as a carbon support was impregnated with 286.4 g of the Pd/Ag/Re solution and allowed to stand for 5.75 hours. Then, the mixture was dried in an oven at 120°C overnight to yield a catalyst comprising 3.3 wt% Pd, 3.2 wt% Ag and 6.6 wt% Re on the carbon support (ACL 40).

### N-2) Production of 1,4-butanediol

Succinic acid was hydrogenated with hydrogen in water in the presence of the catalyst comprising palladium, silver and rhenium metals on the carbon support under pressure of 2500 psig at 160 °C, a GHSV of 2760 hr⁻¹ and a LHSV of 0.55 hr⁻¹ to yield 1,4-butanediol.
silverr

| | | | |
|---|---|---|---|
| | | | |
| | | GBL | THF |
| Pt/M3F-41 | 52.6 | 23.1 | 14.4 |
| Pd/MCM-41 | 50.7 | 23.9 | 15.1 |
| Ru/MCM-41 | 62.5 | 30.3 | 18.8 |

### Example 16: Production of gamma-butyrolactone from 1,4-butanediol

The 1,4-butanediol produced in Example 14 was dehydrogenated in the presence of a copper-zinc-based catalyst to yield gamma-bytyrolactone.

### 16-1) Preparation of catalyst

In a flask, 195 g of copper acetate, 20 g of zinc nitrate, 101 g of aluminum nitrate and 36 g of zirconyl nitrate were dissolved in water. To the solution, a solution of 124 g of sodium hydroxide in 1 L of water was added to form a precipitate by co-precipitation. The precipitate was washed with water, dried, and then calcined at 500 °C to yield a catalyst precursor. 25 g of the catalyst precursor was charged into a catalyst bed (inner diameter: 17 mm; length: about 100 mm) of a fixed-bed atmospheric pressure vapor-phase circulating reactor and reduced with hydrogen (diluted with nitrogen) as a reducing agent at 200°C for 8 hours to establish a Cu-ZnO-Al₂O₃-ZrO₃ catalyst layer for production of gamma-butyrolactone in the reactor.

### 16-2) Production of gamma-butyrolactone

The carrier gas nitrogen was introduced downward into the fixed-bed atmospheric pressure vapor-phase circulating reactor having the Cu-ZnO-Al₂O₃-ZrO₃ catalyst layer at a flow rate of 30 ml/min. 1,4-butanediol was supplied together with the nitrogen gas, vaporized in the vaporizing layer and supplied to the catalyst layer. Herein, the temperature of the vaporizing layer and the catalyst layer was 240 °C. The percent change in the LHSV (liquid hourly space velocity) of 1,4-butanediol, gamma-butyrolactone selectivity and gamma-butyrolactone yield were 99.9%, 98% and 97.9%, respectively.

### Example 17: Production of tetrahydrofuran from 1,4-butanediol

The 1,4-butanediol produced in Example N was dehydrated in the presence of a tungstic oxide catalyst supported on an alumina carrier to produce tetrahydrofuran.

Specifically, 150 g of 1,4-butanediol and 15.0 g of tungstic acid (H₂WO₄) were charged into an autoclave and heated under hydrogen pressure of 1000 psi at 200 °C with stirring at 1000 rpm for 2 hours to produce 112 g of tetrahydrofuran.

### [Accession Number]

Name of Depositary authority : Korean Culture Center of Microorganisms (INTERNATIONAL)
Accession Number : KCCM10568
Name of Depositary authority : Korean Culture Center of Microorganisms (INTERNATIONAL)
Accession Number : KCCM10872
Date of Deposit : 2007.07.05
Name of Depositary authority : Korean Culture Center of Microorganisms (INTERNATIONAL)
Accession Number : KCCM10767
Name of Depositary authority : Korean Culture Center of Microorganisms (INTERNATIONAL)
Accession Number : KCCM10873
Date of Deposit : 2007.07.05

<110> CJ CheilJedang Corporation
<120> The method for producing bio-based homoserinelactone and bio-based
   organic acid from O-acylhomoserine by microorganism
<130> PA12-0380
<160> 16
<170> KopatentIn 2.0
<210> 1
   <211> 70
   <212> DNA
   <213> Artificial Sequence
<220>
   <223> primer
<400> 1
   ttactctggt gcctgacatt tcaccgacaa agcccaggga acttcatcac gtgtaggctg      60
   gagctgcttc                                                              70
<210> 2
   <211> 70
   <212> DNA
   <213> Artificial Sequence
<220>
   <223> primer
<400> 2
   ttaccccttg tttgcagccc ggaagccatt ttccaggtcg gcaattaaat catatgaata      60
   tcctccttag                                                              70
<210> 3
   <211> 70
   <212> DNA
   <213> Artificial Sequence
<220>
   <223> primer
<400> 3
   aaagaatatg ccgatcggtt cgggcttagg ctccagtgcc tgttcggtgg gtgtaggctg      60
   gagctgcttc                                                              70
<210> 4
   <211> 70
   <212> DNA
   <213> Artificial Sequence
<220>
   <223> primer
<400> 4
   agacaaccga catcgctttc aacattggcg accggagccg ggaaggcaaa catatgaata 60
   tcctccttag 70
<210> 5
   <211> 71
   <212> DNA
   <213> Artificial Sequence
<220>
   <223> primer
<400> 5
   atggctgaat ggagcggcga atatatcagc ccatacgctg agcacggcaa ggtgtaggct 60
   ggagctgctt c 71
<210> 6
   <211> 65
   <212> DNA
   <213> Artificial Sequence
<220>
   <223> primer
<400> 6
   gtattcccac gtctccgggt taatccccat ctcacgcatg atctccatat gaatatcctc 60
   cttag 65
<210> 7
   <211> 19
   <212> DNA
   <213> Artificial Sequence
<220>
   <223> primer
<400> 7
   gggctttgtc ggtgaaatg **19**
<210> 8
   <211> 19
   <212> DNA
   <213> Artificial Sequence
<220>
   <223> primer
<400> 8
   actttgcgat gagcgagag **19**
<210> 9
   <211> 28
   <212> DNA
   <213> Artificial Sequence
<220>
   <223> primer
<400> 9
   aatggatcct gccgtgagcg gcgaatac 28
<210> 10
   <211> 28
   <212> DNA
   <213> Artificial Sequence
<220>
   <223> primer
<400> 10
   agctctagac tgctgaggta cgtttcgg 28
<210> 11
   <211> 23
   <212> DNA
   <213> Artificial Sequence
<220>
   <223> primer
<400> 11
   catatgccta cctccgaaca gaa 23
<210> 12
   <211> 26
   <212> DNA
   <213> Artificial Sequence
<220>
   <223> primer
<400> 12
   aagctttcaa aggaaaactc cttcgt 26
<210> 13
   <211> 20
   <212> DNA
   <213> Artificial Sequence
<220>
   <223> primer
<400> 13
   catatgccca ccctcgcgcc 20
<210> 14
   <211> 25
   <212> DNA
   <213> Artificial Sequence
<220>
   <223> primer
<400> 14
   aagcttttag atgtagaact cgatg 25
<210> 15
   <211> 70
   <212> DNA
   <213> Artificial Sequence
<220>
   <223> primer
<400> 15
   caatttcttg cgtgaagaaa acgtctttgt gatgacaact tctcgtgcgt gtgtaggctg 60
   gagctgcttc 70
<210> 16
   <211> 70
   <212> DNA
   <213> Artificial Sequence
<220>
   <223> primer
<400> 16
   aatccagcgt tggattcatg tgccgtagat cgtatggcgt gatctggtag catatgaata 60
   tcctccttag 70

## Claims

1. A method for producing homoserine lactone and organic acid, comprising
- producing O-acyl homoserine by a microorganism, and
- hydrolysing the O-acyl homoserine in the presence of hydrochloric acid, wherein O-acyl homoserine and hydrochloric acid are used at a molar ratio of 1: 1-15.

2. The method according to claim 1, wherein the O-acyl homoserine includes O-acetyl-L-homoserine or O-succinyl-L-homoserine.

3. The method according to claim 1, wherein the organic acid includes acetic acid or succinic acid.

4. The method according to claim 1, wherein the microorganism is a strain whose cystathionine gamma synthase, O-succinylhomoserine sulfhydrylase or O-acetylhomoserine sulfhydrylase activity was removed or weakened.

5. The method according to claim 2, wherein the microorganism is a strain whose homoserine O-acetyl transferase activity was enhanced.

6. The method according to claim 2, wherein the microorganism is a strain whose homoserine O-succinyl transferase activity was enhanced.

7. A method for producing gamma-butyrolactone, comprising the steps of:
- producing O-acyl homoserine by a microorganism;
- producing homoserine lactone and organic acid through hydrolysis of the O-acyl homoserine in the presence of hydrochloric acid, wherein O-acyl homoserine and hydrochloric acid are used at a molar ratio of 1: 1-15; and
deaminating the homoserine lactone by hydrodenitrification in the presence of a metal catalyst and hydrogen gas to produce gamma-butyrolactone.

8. A method as claimed in claim 7, further comprising the step of etherifying the gamma-butyrolactone in the presence of an indium bromide catalyst and a silane compound to produce tetrahydrofuran.

9. A method as claimed in claim 7, further comprising the step of producing 2-pyrrolidone from the gamma-butyrolactone in the presence of an aqueous ammonia solution.

10. A method as claimed in claim 7, further comprising the step of producing N-methyl-2-pyrrolidone from the gamma-butyrolactone in the presence of liquid methylamine.

11. A method as claimed in claim 7, further comprising the steps of:
- dehydrating the gamma-butyrolactone in the presence of liquid ethanolamine to produce N-(2-hydroxyethyl)-2-pyrrolidone (first-stage reaction); and
- dehydrating the N-(2-hydroxyethyl)-2-pyrrolidone in the presence of an oxide catalyst containing an alkali metal or an alkaline earth metal and silicon to produce N-vinyl-2-pyrrolidone (second-stage reaction).

12. A method as claimed in claim 7, further comprising the step of hydrogenating the gamma-butyrolactone in the presence of a ruthenium catalyst with an imidazole ligand to produce 1,4-butanediol.

13. A method for producing ethanol, comprising the steps of:
- producing O-acetyl-L-homoserine by a microorganism;
- producing homoserine lactone and acetic acid through hydrolysis of the O-acetyl-L-homoserine in the presence of hydrochloric acid, wherein O-acetyl-L-homoserine and hydrochloric acid are used at a molar ratio of 1: 1-15; and
- hydrogenating the acetic acid in the presence of a catalyst comprising a metal, a siliceous support and at least one support modifier to produce ethanol, wherein the support modifier is selected from an oxide or metasilicate of sodium, potassium, magnesium, calcium, scandium, yttrium or zinc.

14. A method as claimed in claim 13, further comprising the step of dehydrating the ethanol in the presence of a zeolite catalyst to produce ethylene.

15. A method as claimed in claim 13, further comprising the steps of
- dehydrating the ethanol in the presence of a catalyst to produce ethylene; and
- polymerizing the ethylene in the presence of a Ziegler-Natta catalyst to produce polyethylene.

16. A method as claimed in claim 13, further comprising the step of hydrolyzing the ethanol in the presence of a platinum-based catalyst to produce monoethylene glycol.

17. A method for producing 1,4-butanediol, comprising the steps of:
- producing O-succinyl-L-homoserine by a microorganism; and
- producing homoserine lactone and succinic acid through hydrolysis of the O-succinyl-L-homoserine in the presence of hydrochloric acid, wherein O-succinyl-L-homoserine and hydrochloric acid are used at a molar ratio of 1: 1-15; and
- hydrogenating the succinic acid in the presence of a metal catalyst on a carbon support to produce 1,4-butanediol.

18. The method according to claim 17, wherein tetrahydrofuran is produced as a byproduct in the step of hydrogenating the succinic acid in the presence of the metal catalyst on the carbon support to produce 1,4-butanediol.

19. A method as claimed in claim 17, further comprising the step of dehydrogenating the 1,4-butanediol in the presence of a copper-zinc-based catalyst to produce gamma-butyrolactone.

20. A method as claimed in claim 17, further comprising the step of dehydrating the 1,4-butanediol in the presence of a catalyst selected from inorganic acid, tungstic oxide and iron phosphate to produce tetrahydrofuran.

21. A method as claimed in claim 17, further comprising the steps of:
- dehydrogenating the 1,4-butanediol in the presence of a copper-zinc-based catalyst to produce gamma-butyrolactone; and
- dehydrating the gamma-butyrolactone with liquid methylamine to produce N-methyl-2-pyrrolidone.

## Patentansprüche

1. Verfahren zum Herstellen von Homoserinlacton und organischer Säure, umfassend
- Herstellen von O-Acylhomoserin durch einen Mikroorganismus und
- Hydrolysieren des O-Acylhomoserins in Gegenwart von Salzsäure, wobei O-Acylhomoserin und Salzsäure in einem Molverhältnis von 1: 1-15 verwendet werden.

2. Verfahren nach Anspruch 1, wobei O-Acylhomoserin O-Acetyl-L-homoserin oder O-Succinyl-L-homoserin einschließt.

3. Verfahren nach Anspruch 1, wobei die organische Säure Essigsäure oder Bernsteinsäure einschließt.

4. Verfahren nach Anspruch 1, wobei der Mikroorganismus ein Stamm ist, dessen Cystathionin-Gamma-Synthase-, O-Succinylhomoserinsulfhydrylase- oder O-Acetylhomoserinsulfhydrylase-Aktivität entfernt oder abgeschwächt wurde.

5. Verfahren nach Anspruch 2, wobei der Mikroorganismus ein Stamm ist, dessen Homoserin-O-acetyltransferase-Aktivität verstärkt wurde.

6. Verfahren nach Anspruch 2, wobei der Mikroorganismus ein Stamm ist, dessen Homoserin-O-succinyltransferase-Aktivität verstärkt wurde.

7. Verfahren zum Erzeugen von Gamma-Butyrolacton, umfassend die folgenden Schritte:
- Herstellen von O-Acylhomoserin durch einen Mikroorganismus;
- Herstellen von Homoserinlacton und organischer Säure durch Hydrolyse des O-Acylhomoserins in Gegenwart von Salzsäure, wobei O-Acylhomoserin und Salzsäure in einem Molverhältnis von 1: 1-15 verwendet werden; und
Deaminieren des Homoserinlactons durch Hydrodenitrifizierung in Gegenwart eines Metallkatalysators und Wasserstoffgas, um Gamma-Butyrolacton herzustellen.

8. Verfahren nach Anspruch 7, weiter umfassend den Schritt des Veretherns des Gamma-Butyrolactons in Gegenwart eines Indiumbromid-Katalysators und einer Silanverbindung, um Tetrahydrofuran herzustellen.

9. Verfahren nach Anspruch 7, weiter umfassend den Schritt des Herstellens von 2-Pyrrolidon aus dem Gamma-Butyrolacton in Gegenwart einer wässrigen Ammoniaklösung.

10. Verfahren nach Anspruch 7, weiter umfassend den Schritt des Herstellens von N-Methyl-2-pyrrolidon aus dem Gamma-Butyrolacton in Gegenwart von flüssigem Methylamin.

11. Verfahren nach Anspruch 7, weiter umfassend die folgenden Schritte:
- Dehydratisieren des Gamma-Butyrolactons in Gegenwart von flüssigem Ethanolamin, um N-(2-Hydroxyethyl)-2-pyrrolidon herzustellen (Reaktion des ersten Stadiums); und
- Dehydratisieren des N-(2-Hydroxyethyl)-2-pyrrolidons in Gegenwart eines ein Alkalimetall oder ein Erdalkalimetall enthaltenden Oxidkatalysators und Silizium, um N-Vinyl-2-pyrrolidon herzustellen (Reaktion des zweiten Stadiums).

12. Verfahren nach Anspruch 7, weiter umfassend den Schritt des Hydrierens des Gamma-Butyrolactons in Gegenwart eines Rutheniumkatalysators mit einem Imidazolliganden, um 1,4-Butandiol herzustellen.

13. Verfahren zum Herstellen von Ethanol, umfassend die folgenden Schritte:
- Herstellen von O-Acetyl-L-homoserin durch einen Mikroorganismus;
- Herstellen von Homoserinlacon und Essigsäure durch Hydrolyse des O-Acetyl-L-homoserins in Gegenwart von Salzsäure, wobei O-Acetyl-L-homoserin und Salzsäure in einem Molverhältnis von 1: 1-15 verwendet werden; und
- Hydrieren der Essigsäure in Gegenwart eines Katalysators, der ein Metall umfasst, eines Siliziumdioxid-Trägermaterials und mindestens eines Trägermaterialmodifizierers, um Ethanol herzustellen, wobei der Trägermaterialmodifizierer aus einem Oxid oder Metasilikat von Natrium, Kalium, Magnesium, Kalzium, Scandium, Yttrium oder Zink ausgewählt ist.

14. Verfahren nach Anspruch 13, weiter umfassend den Schritt des Dehydratisierens des Ethanols in Gegenwart eines Zeolith-Katalysators, um Ethylen herzustellen.

15. Verfahren nach Anspruch 13, weiter umfassend die folgenden Schritte:
- Dehydratisieren des Ethanols in Gegenwart eines Katalysators, um Ethylen herzustellen; und
- Polymerisieren des Ethylens in Gegenwart eines Ziegler-Natta-Katalysators, um Polyethylen herzustellen.

16. Verfahren nach Anspruch 13, weiter umfassend den Schritt des Hydrolysierens des Ethanols in Gegenwart eines Katalysators auf Platinbasis, um Monoethylenglycol herzustellen.

17. Verfahren zum Herstellen von 1,4-Butandiol, umfassend die folgenden Schritte:
- Herstellen von O-Succinyl-L-homoserin durch einen Mikroorganismus; und
- Herstellen von Homoserinlacton und Bernsteinsäure durch Hydrolyse des O-Succinyl-L-homoserins in Gegenwart von Salzsäure, wobei O-Succinyl-L-homoserin und Salzsäure in einem Molverhältnis von 1: 1-15 verwendet werden; und
- Hydrieren der Bernsteinsäure in Gegenwart eines Metallkatalysators auf einem Kohlenstoffträgermaterial, um 1,4-Butandiol herzustellen.

18. Verfahren nach Anspruch 17, wobei beim Schritt des Hydrierens der Bernsteinsäure in Gegenwart des Metallkatalysators auf dem Kohlenstoffträgermaterial zur Herstellung von 1,4-Butandiol Tetrahydrofuran als Nebenprodukt hergestellt wird.

19. Verfahren nach Anspruch 17, weiter umfassend den Schritt des Dehydrierens des 1,4-Butandiols in Gegenwart eines Katalysators auf Kupfer-Zink-Basis, um Gamma-Butyrolacton herzustellen.

20. Verfahren nach Anspruch 17, weiter umfassend den Schritt des Dehydratisierens des 1,4-Butandiols in Gegenwart eines Katalysators, ausgewählt aus anorganischer Säure, Wolframoxid und Eisenphosphat, um Tetrahydrofuran herzustellen.

21. Verfahren nach Anspruch 17, weiter umfassend die folgenden Schritte:
- Dehydrieren des 1,4-Butandiols in Gegenwart eines Katalysators auf Kupfer-Zink-Basis, um Gamma-Butyrolacton herzustellen; und
- Dehydratisieren des Gamma-Butyrolactons mit flüssigem Methylamin, um N-Methyl-2-pyrrolidon herzustellen.

## Revendications

1. Procédé de production d'homosérine lactone et d'acide organique, comprenant
- la production d'O-acyl homosérine par un microorganisme, et
- l'hydrolysation de l'O-acyl homosérine en présence d'acide chlorhydrique, dans lequel l'O-acyl homosérine et l'acide chlorhydrique sont utilisés à un rapport molaire de 1:1-15.

2. Procédé selon la revendication 1, dans lequel l'O-acyl homosérine comprend l'O-acétyl-L-homosérine ou l'O-succinyl-L-homosérine.

3. Procédé selon la revendication 1, dans lequel l'acide organique comprend l'acide acétique ou l'acide succinique.

4. Procédé selon la revendication 1, dans lequel le microorganisme est une souche dont l'activité de la cystathionine gamma-synthase, de l'O-succinylhomosérine sulfhydrylase ou de l'O-acétylhomosérine sulfhydrylase a été éliminée ou affaiblie.

5. Procédé selon la revendication 2, dans lequel le microorganisme est une souche dont l'activité de l'homosérine O-acétyl transférase a été augmentée.

6. Procédé selon la revendication 2, dans lequel le microorganisme est une souche dont l'activité de l'homosérine O-succinyl transférase a été augmentée.

7. Procédé de production de gamma-butyrolactone, comprenant les étapes de :
- production d'O-acyl homosérine par un microorganisme ;
- production d'homosérine lactone et d'acide organique par l'hydrolyse de l'O-acyl homosérine en présence d'acide chlorhydrique, dans lequel l'O-acyl homosérine et l'acide chlorhydrique sont utilisés dans un rapport molaire de 1:1-15 ; et
désamination de l'homosérine lactone par hydrodénitrification en présence d'un catalyseur métallique et d'hydrogène gazeux pour produire de la gamma-butyrolactone.

8. Procédé selon la revendication 7, comprenant en outre l'étape d'éthérification de la gamma-butyrolactone en présence d'un catalyseur de bromure d'indium et d'un composé de silane pour produire du tétrahydrofurane.

9. Procédé selon la revendication 7, comprenant en outre l'étape de production de 2-pyrrolidone à partir de la gamma-butyrolactone en présence d'une solution d'ammoniaque aqueux.

10. Procédé selon la revendication 7, comprenant en outre l'étape de production de N-méthyl-2-pyrrolidone à partir de la gamma-butyrolactone en présence de méthylamine liquide.

11. Procédé selon la revendication 7, comprenant en outre les étapes de :
- déshydratation de la gamma-butyrolactone en présence d'éthanolamine liquide pour produire de la N-(2- hydroxyéthyl)-2-pyrrolidone (première étape réactionnelle) ; et
- déshydratation de la N-(2-hydroxyéthyl)-2-pyrrolidone en présence d'un catalyseur d'oxyde contenant un métal alcalin ou un métal alcalino-terreux et de silicium pour produire de la N-vinyl-2-pyrrolidone (seconde étape réactionnelle).

12. Procédé selon la revendication 7, comprenant en outre l'étape d'hydrogénation de la gamma-butyrolactone en présence d'un catalyseur de ruthénium avec un ligand d'imidazole pour produire du 1,4-butanediol.

13. Procédé de production d'éthanol, comprenant les étapes de :
- production d'O-acétyl-L-homosérine par un microorganisme ;
- production d'homosérine lactone et d'acide acétique par l'hydrolyse de l'O-acétyl-L-homosérine en présence d'acide chlorhydrique, dans lequel l'O-acétyl-L-homosérine et l'acide chlorhydrique sont utilisés dans un rapport molaire de 1:1-15 ; et
- hydrogénation de l'acide acétique en présence d'un catalyseur comprenant un métal, un support siliceux et au moins un modificateur de support pour produire de l'éthanol, dans lequel le modificateur de support est sélectionné parmi un oxyde ou métasilicate de sodium, potassium, magnésium, calcium, scandium, yttrium ou zinc.

14. Procédé selon la revendication 13, comprenant en outre l'étape de déshydratation de l'éthanol en présence d'un catalyseur de zéolite pour produire de l'éthylène.

15. Procédé selon la revendication 13, comprenant en outre les étapes de
- déshydratation de l'éthanol en présence d'un catalyseur pour produire de l'éthylène ; et
- polymérisation de l'éthylène en présence d'un catalyseur Ziegler-Natta pour produire du polyéthylène.

16. Procédé selon la revendication 13, comprenant en outre l'étape d'hydrolysation de l'éthanol en présence d'un catalyseur à base de platine pour produire du monoéthylène glycol.

17. Procédé de production de 1,4-butanediol, comprenant les étapes de :
- production d'O-succinyl-L-homosérine par un microorganisme ; et
- production d'homosérine lactone et d'acide succinique par hydrolyse de l'O-succinyl-L-homosérine en présence d'acide chlorhydrique, dans lequel l'O-succinyl-L-homosérine et l'acide chlorhydrique sont utilisés à un rapport molaire de 1:1-15 ; et
- hydrogénation de l'acide succinique en présence d'un catalyseur métallique sur un support de carbone pour produire du 1,4-butanediol.

18. Procédé selon la revendication 17, dans lequel le tétrahydrofurane est produit en tant que sous-produit dans l'étape d'hydrogénation de l'acide succinique en présence du catalyseur métallique sur le support de carbone pour produire du 1,4-butanediol.

19. Procédé selon la revendication 17, comprenant en outre l'étape de déshydrogénation du 1,4- butanediol en présence d'un catalyseur à base de cuivre-zinc pour produire de la gamma-butyrolactone.

20. Procédé selon la revendication 17, comprenant en outre l'étape de déshydratation du 1,4- butanediol en présence d'un catalyseur sélectionné parmi un acide inorganique, l'oxyde tungstique et le phosphate de fer pour produire du tétrahydrofurane.

21. Procédé selon la revendication 17, comprenant en outre les étapes de :
- déshydrogénation du 1,4-butanediol en présence d'un catalyseur de cuivre-zinc pour produire de la gamma-butyrolactone ; et
- déshydratation de la gamma-butyrolactone avec de la méthylamine liquide pour produire de la N-méthyl-2- pyrrolidone.
